(19) Europäisches Patentamt
European Patent Office
Office européen des brevets

(11) Numéro de publication: **0 211 744 B1**

(12) **FASCICULE DE BREVET EUROPEEN**

(45) Date de publication de fascicule du brevet: **04.12.91**

(51) Int. Cl.$^5$: **C07K 5/00**, A61K 37/64

(21) Numéro de dépôt: **86401637.3**

(22) Date de dépôt: **22.07.86**

Le dossier contient des informations techniques présentées postérieurement au dépôt de la demande et ne figurant pas dans le présent fascicule.

(54) **Dérivés aminoalcools peptidiques contenant un atome de carbone tetrasubstitué inhibiteurs de la rénine et des protéases acides - Procédé d'obtention et compositions pharmaceutiques.**

(30) Priorité: **31.07.85 FR 8511728**

(43) Date de publication de la demande: **25.02.87 Bulletin 87/09**

(45) Mention de la délivrance du brevet: **04.12.91 Bulletin 91/49**

(84) Etats contractants désignés: **AT BE CH DE FR GB IT LI LU NL SE**

(56) Documents cités:
**EP-A- 0 081 783**
**EP-A- 0 111 266**
**EP-A- 0 156 319**
**EP-A- 0 156 321**
**EP-A- 0 156 322**
**EP-A- 0 161 588**

(73) Titulaire: **SANOFI**
**40, Avenue George V**
**F-75008 Paris(FR)**

Titulaire: **INSTITUT NATIONAL DE LA SANTE ET DE LA RECHERCHE MEDICALE (INSERM)**
**101, rue de Tolbiac**
**F-75654 Paris Cédex 13(FR)**

(72) Inventeur: **Wagnon, Jean**
**Villa 34 - avenue du Pont Trinquat**
**F-34000 Montpellier(FR)**
Inventeur: **Guegan, Remy**
**355, chemie du Thym**
**F-34I70 Castelnau Le Lez(FR)**
Inventeur: **Lacour, Colette**
**Lotissement les Rêves Rue de Braine**
**F-34I00 Montpellier(FR)**
Inventeur: **Nisato, Dino**
**4, Impasse de l'Olivette**
**F-34680 Saint Georges D'Orques(FR)**

(74) Mandataire: **Gillard, Marie-Louise et al**
**Cabinet Beau de Loménie 55, Rue d'Amsterdam**
**F-75008 Paris(FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention concerne des nouveaux dérivés peptidiques inhibiteurs de la rénine et plus généralement des protéases acides. Elle concerne également un procédé pour leur obtention et leur application en thérapeutique.

En 1970, UMEZAWA a isolé, à partir d'une culture de Streptomyces, un pentapeptide désigné sous le nom de pepstatine dont la structure a éte établie ultérieurement et répond à la formule :
Isovaléryl - L-valyl - L-valyl - Statyl - L-alanyl - Statine
dans laquelle on désigne sous le nom de "statine" un acide aminé non usuel, l'acide (3S, 4S) amino-4 hydroxy-3 méthyl-6 heptanoïque.

Il a été montré que la pepstatine est un inhibiteur de protéases acides et agit en particulier sur la pepsine, la cathepsine D et la rénine. Spécialement, la rénine, enzyme d'origine rénale, intervient dans la séquence angiotensinogène, angiotensine I, angiotensine II au niveau de la transformation de l'angiotensinogène en angiotensine.

L'angiotensine II étant un puissant agent vasoconstricteur, intervient dans la régulation de la pression artérielle. On a envisagé d'utiliser la pepstatine pour lutter contre l'hypertension artérielle chez l'homme. Toutefois, la pepstatine agissant sur l'ensemble des protéases acides et présentant une faible solubilité en milieux aqueux et une faible affinité pour la rénine, son emploi en thérapeutique s'est avéré difficile. Des dérivés de la pepstatine ont été décrits dans la littérature scientifique. Par exemple, on a tenté de solubiliser la pepstatine par allongement de la chaîne peptidique (J. Cardiovasc. Pharmacol., 1980, 2, 687-698).

Selon la présente invention on a trouvé qu'il est possible d'obtenir des produits très actifs sans allonger la chaîne peptidique mais en introduisant des résidus hydrophiles sur différents sites d'un peptide analogue de la pepstatine. Ceci est d'autant plus surprenant que l'art antérieur (brevet Merck n° US 4470971) enseigne que la présence de substituants hydrophiles diminue l'activité inhibitrice envers la rénine.

La présente invention pour objet des peptides présentant un niveau élevé d'activité en tant qu'inhibiteurs de la rénine et d'autres protéases acides.

Dans la présente description et dans les revendications les abréviations suivantes seront utilisées.

Acides aminés et groupes protecteurs ou activateurs.

Ces abréviations sont en accord avec celles indiquées par la Commission de Nomenclature de l'IUPAC-IUB section Biochimie. Les recommandations les plus récentes sont rapportées dans Eur. J. Biochem., 1984, 138, 5-7 et 9-37.

Acides aminés :

| | |
|---|---|
| Abu | : Acide alpha aminobutyrique |
| Ala | : Alanine |
| Asn | : Asparagine |
| Asp | : Acide Aspartique |
| Cpg | : Cyclopentylglycine |
| Gln | : Glutamine |
| Gly | : Glycine |
| His | : Histidine |
| Ile | : Isoleucine |
| Leu | : Leucine |
| Met | : Methionine |
| Nle | : Norleucine |
| Nva | : Norvaline |
| Phe | : Phénylalanine |
| Phg | : Phenylglycine |
| Ser | : Sérine |
| Val | : Valine |
| (tauMe)His | : (télé-méthyl)Histidine |

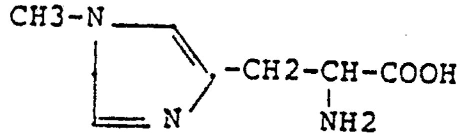

(Pyr-3)Ala : (Pyridyl-3) Alanine

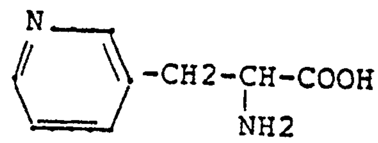

(Benzimidazolyl-2)Ala :

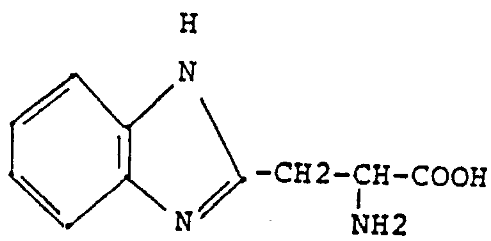

Ces acides aminés sont de configuration L.
Sta : Statine : acide amino-4 hydroxy-3 méthyl-6 heptanoïque
AHPPA : acide amino-4 hydroxy-3 phényl-5 pentanoïque
ACHPA : acide amino-4 cyclohexyl-5 hydroxy-3 pentanoïque
Sta, AHPPA et ACHPA sont, sauf indication contraire, de configuration 3S, 4S.

Groupes protecteurs et activateurs :

| | |
|---|---|
| Ac | : Acétyle |
| Boc | : tert.butyloxycarbonyle |
| $(Boc)_2$ O | : Anhydride bis terbutyloxycarbonique |
| HONSu | : N-hydroxysuccinimide |
| OEt | : Ester éthylique |
| OMe | : Ester méthylique |
| ONp | : Ester p nitrophénylique |
| ONSu | : Ester de N-hydroxysuccinimide |
| i Va | : Isovaléryle |
| Z | : Benzyloxycarbonyle |

De plus les abréviations suivantes sont éqalement utilisées :

| | |
|---|---|
| AcOEt | : Acétate d'éthyle |
| AcOH | : Acide acétique |
| Bop | : Hexafluorophosphate de benzyloxy tris diméthylamino phosphonium |
| CCM | : Chromatographie en couche mince |
| DCCI | : Dicyclohexylcarbodiimide |
| DCHA | : Dicyclohexylamine |
| DCU | : Dicyclohexylurée |
| DIPEA | : Diisopropyléthylamine |
| DMF | : Diméthylformamide |
| DMSO | : Diméthylsulfoxyde |
| Ether | : Ether éthylique |
| HOBt | : Hydroxy-1 benzotriazole |
| MeOH | : Méthanol |

| | |
|---|---|
| NEM | : N-éthyl morpholine |
| NMM | : N-méthyl morpholine |
| TA | : Température ambiante |
| TFA | : Acide trifluoracétique |
| Sta réduite | : Amino-4 méthyl-6 heptane-1,3 diol. |

"on débocule" signifie : on enlève le groupement protecteur Boc.

Les carbones asymétriques pour lesquels la configuration est déterminée sont notés

```
*
C
```

La présente invention a donc pour objet les composés répondant à la formule générale suivante :

```
R1-NH-CH-CO-NH-CH-CO-NH-CH-CHOH-CH2-CO-X-N-R5
      |        |        |                 |
      R2       R3       CH2               R4
                        |
                        Q                        (I)
```

dans laquelle :

- R1 représente un groupe acyle choisi parmi les groupes alkylcarbonyle, alcoxycarbonyle, arylcarbonyle, arylalkylcarbonyle, arylalcoxycarbonyle, hétérocyclylcarbonyle, hétérocyclyalkylcarbonyle dans lequel le groupe alkyle est éventuellement substitué par un groupe hydroxy, hétérocyclylcarbonylalkylcarbonyle, hétérocyclylalcénylcarbonyle, cycloalkylcarbonyle ou un groupe (alkyle inférieur)-sulfonyle non substitué ou substitué sur l'alkyle par un groupe amino libre ou portant un groupe protecteur ou par un phényle ; ou un groupe phénylsulfonyle non substitué ou substitué sur le noyau phényle par un alkyle inférieur ;
- R2 représente un groupe alkyle inférieur non substitué ou substitué par un phényle, naphtyle, cyclohexyle, ou pyridyle ou R2 représente un radical phényle, naphtyle, cyclohexyle ou pyridyle ;
- R3 représente l'hydrogène, un alcényle inférieur, un phényle, un naphtyle, un cycloalkyle contenant 3 à 6 atomes de carbone, un groupe hétérocyclique monocyclique à 5 ou 6 chaînons non substitué ou substitué avec un alkyle inférieur ou trifluorométhyle, ou bien un alkyle inférieur non substitué ou substitué par un groupe amino libre ou portant un groupe protecteur, par un groupe di(alkyle inférieur)amino, par un carboxyle libre ou estérifié avec un alkyle inférieur ou un benzyle, par un carbamoyle libre ou substitué avec un ou deux alkyles inférieurs ou avec un phényle, par un groupe hydroxy, alcoxy inférieur ou benzyloxy, par un groupe pyridylméthyloxy, par un groupe alkylthio inférieur, (alkyle inférieur)sulfinyle ou (alkyle inférieur)sulfonyle, par un phényle non substitué ou substitué par un hydroxyle, par un naphtyle, par un cycloalkyle contenant de 3 à 6 atomes de carbone, par un groupe hétérocyclique monocyclique à 5 ou 6 chaînons non substitué ou substitué avec un alkyle inférieur ou un trifluorométhyle ou par un groupe hétérocyclique bicyclique non substitué ou substitué avec un alkyle inférieur ou un trifluorométhyle ;
- Q représente l'isopropyle, le phényle ou le cyclohexyle formant respectivement avec le radical

```
-NH-CH-CHOH-CH2-CO
     CH2
```

le résidu de l'aminoacide Sta, ou AHPPA ou ACHPA ;

- X est soit une liaison directe, soit le résidu d'un aminoacide tel que Ala, Nva, Val, Leu, Nle, Ile, Phg, Abu, Cpg ;
- R4 représente l'hydrogène ou un alkyle inférieur non substitué ou substitué par un radical indolyle, pyridyle, imidazolyle ou phényle ;

- R5 représente un groupe hydrocarboné aliphatique ou cyclique de 3 à 2O atomes de carbone non substitué ou substitué par un groupe phényle, hydroxyphényle ou amino, ledit groupe hydrocarboné contenant simultanément au moins un groupe hydroxyle et au moins un atome de carbone ne présentant pas de liaison C-H ; ainsi que leurs sels éventuels pharmaceutiquement acceptables avec des acides minéraux ou organiques ou des métaux alcalins ou alcalino-terreux.

Les produits préférés selon la présente invention ont la formule (I) ci-dessus dans laquelle R5 représente :

a) un groupe

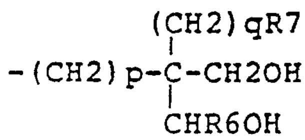

où :

- p représente un nombre entier compris entre zéro et 5,
- q représente un nombre entier compris entre O et 5,
- R6 représente l'hydrogène ou un alkyle inférieur, et
- R7 représente l'hydrogène, un groupement hydroxyle, un groupement amine, un groupement carboxy, un groupement pyridyle ou un groupement phényle non substitué ou substitué par un hydroxyle ; à la condition que lorsque q = O, R7 est différent de l'hydrogène

b) un groupe

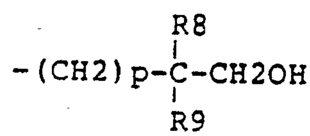

- p est tel que défini ci-dessus ;
- R8 représente un alkyle inférieur ;
- R9 représente un alkyle inférieur non substitué ou substitué par un groupe amino libre ou portant un groupe protecteur ;
- ou R8 et R9 ensemble forment avec le carbone auquel ils sont reliés un cycloalkyle de 3 à 8 atomes de carbone.

Les composés de l'invention contiennent de préférence de 1 à 3 groupes hydroxyle dans le radical R5. Particulièrement préférés sont les produits de formule (I) ci-dessus dans laquelle R5 est groupe choisi parmi :

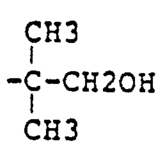 , 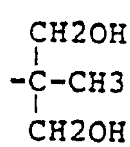 , 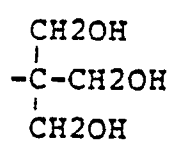 ou 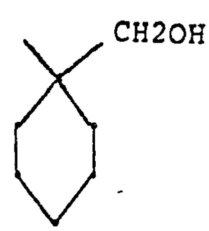

Dans la présente description, le terme "alkyle" désigne les radicaux d'hydrocarbures aliphatiques, saturés ou insaturés contenant de 1 à 10 atomes de carbone, les groupes alkyles préférés sont les alkyles inférieurs tels que définis ci-après.

Les expressions "alkyle inférieur" et "alcényle inférieur", telles qu'utilisées ici, désignent les radicaux d'hydrocarbures aliphatiques saturés ou insaturés contenant jusqu'à 6 atomes de carbone.

Les expressions "alcoxy inférieur" et "alkylthio inférieur" représentent les groupes hydroxyle et thiol substitués par un groupe alkyle inférieur tel que défini ci-dessus.

L'expression "hétérocycle monocyclique à 5 ou 6 chaînons" inclut la pyrrolidine, l'imidazole, le thiazole, le thiophène, le furanne, le pyrrole, le triazole, l'oxazole, l'isoxazole, la pyridine, les thiadiazoles.

L'expression "hétérocycle bicyclique" inclut le benzimidazole, le benzothiazole, le benzoxazole, l'indole

ou leurs homologues hydrogénés.

Par "groupe protecteur" on entend un groupe protecteur utilisé normalement dans la chimie des peptides, par exemple Boc, Z ou iVa.

L'expression "groupe acyle" utilisée pour la définition de R1 inclut les résidus d'acides carboxyliques aliphatiques, alicycliques, aromatiques ou hétérocycliques. Des groupes acyle préférés sont le résidu de l'acide carbonique estérifié, notamment les groupes Boc et Z, le résidu d'acides alcanoïques contenant de 2 à 6 atomes de carbone, notamment le groupe iVa, le résidu de l'acide cyclohexylcarboxylique, le résidu d'acides phénylaliphatiques, notamment les groupes phénylacétyle, 3-phénylpropionyle, dibenzylacétyle, le résidu d'acides arylcarboxyliques, tels que l'acide naphtoïque, biphényl carboxylique et l'acide benzoïque non substitué ou substitué sur le noyau phényle, notamment le groupe benzoyle, le résidu d'un acide carboxylique dont le carboxyle est lié à un hétérocycle monocyclique à 5 ou 6 chaînons, notamment les groupes picolinoyle, nicotinoyle et isonicotinoyle et le résidu d'acides alcanoïques ou alcénoïques, tels que l'acide acétique, l'acide propionique, l'acide butyrique, l'acide valérique et leurs dérivés oméga-hydroxy ou oméga-oxo, substitués en position oméga par un hétérocycle monocyclique à 5 ou 6 chaînons comme exemplifiés ci-dessus.

Plus particulièrement, la présente invention concerne, d'une façon préférentielle, les aminoalcools peptidiques de formule I dans laquelle R2, R3, Q, R4 et R5 sont tels que définis ci-dessus et R1 représente l'un des groupements suivants :

$$(CH3)3C - O - \underset{\underset{O}{\|}}{C} -$$

$$(CH3)2CH - CH2 - \underset{\underset{O}{\|}}{C} -$$

$$C6H5 - (CH2)a - \underset{\underset{O}{\|}}{C} - , \text{ où } a = 0,1 \text{ ou } 2$$

$$(C6H5 - CH2)2CH - \underset{\underset{O}{\|}}{C} -$$

$$C6H5 - CH2 - O - \underset{\underset{O}{\|}}{C} -$$

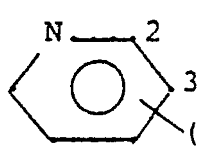

$$(CH2)b - \underset{\underset{O}{\|}}{C} - , \text{ où } b = 0,1,2,3,4,5 \text{ ou } 6$$

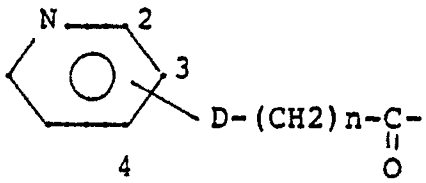 , où D = 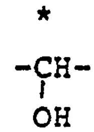 ou 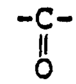

et n = 1,2,3,4 ou 5

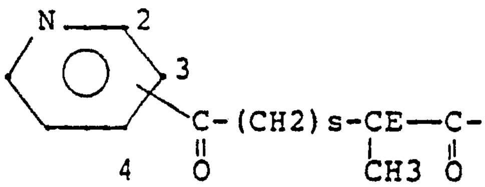 , où E = -H ou -CH3

et s = 1,2,3 ou 4

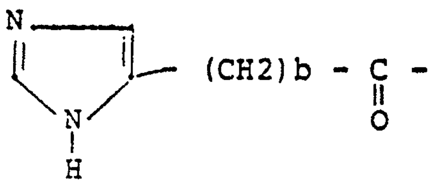 , où b = 0,1,2,3,4,5 ou 6

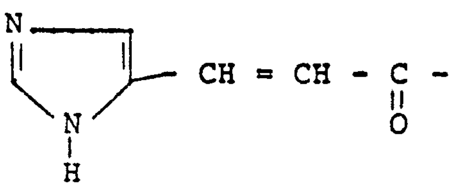

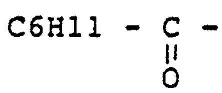

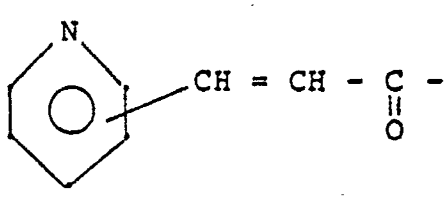

NH2CH2CH2 - SO2-
BocNHCH2CH2 - SO2-
Z-NHCH2CH2 - SO2-
A - SO2 - , où A est un alkyle inférieur
C6H5 - (CH2)a - SO2 -, où a = 0,1 ou 2

Particulièrement préférés sont les aminoalcools peptidiques de formule (I) dans laquelle R2, R3, Q, R4 et R5 sont tels que définis ci-dessus et R1 représente un groupe choisi parmi les groupes suivants :

- isovaleryle,
- (pyridyl-2)-4 oxo-4 butyryle,
- (pyridyl-2)-4 hydroxy-4 butyryle,
- (pyridyl-3)-3 propionyle,
- (pyridyl-3)-4 butyryle,
- nicotinoyle,

- benzènesulfonyle.

De plus sont particulièrement préférés les composés de formule (I) dans lesquels R1, R2, R4, R5 et Q sont tels que définis précédemment et R3 est tel que le résidu NH-CH(R3)-CO- représente le résidu (tauMe)His.

Une autre classe de composés préférés de l'invention est constituée par les composés de formule (I) dans laquelle :

- R1 représente un groupe choisi parmi les groupes suivants : isovaléryle, tert.butyloxycarbonyle, (pyridyl-3)-3 propionyle, (pyridyl-3)-4 butyryle, nicotinoyle ;
- R2 représente un benzyle ;
- R3 représente n-butyle ou un méthyle substitué par un radical hétérocyclique choisi parmi imidazolyl-4, méthyl-1 imidazolyl-4, méthyl-2 imidazolyl-4, pyridyl-3, benzimidazolyl-2 ;
- R4 est l'hydrogène ;
- X représente le résidu de l'un des aminoacides Ile ou Ala ;
- Q et R5 sont tels que définis précédemment.

Les produits selon l'invention peuvent être préparés selon les méthodes habituelles de la chimie des peptides. Ainsi, selon un autre de ses aspects, l'invention se réfère à un procédé pour la préparation des dérivés aminoalcools peptidiques de formule I ci-dessus, qui consiste à traiter un aminoalcool de formule :

```
W - N - R5
    |
    R4
```

dans laquelle W est un groupe protecteur ou l'hydrogène et R4 et R5 sont tels que définis ci-dessus et dont les groupes hydroxyle sont éventuellement protégés, avec l'ester d'alkyle inférieur de l'acide aminé de formule :

```
H-X'-OH   ou   H2N-CH-CHOH-CH2-COOH   protégé,
                   |
                   CH2
                   |
                   Q
```

dans laquelle X' a la définition donnée ci-dessus pour X, mais X' est autre qu'une liaison directe et Q est tel que défini ci-dessus, puis à procéder étape par étape à l'élongation de la chaîne peptidique par couplage avec les acides aminés ou les fragments suivants, convenablement protégés, les différentes opérations étant effectuées en utilisant soit un ester activé de l'aminoacide ou du fragment à coupler, soit l'aminoacide N- protégé en présence de DCCI, les groupes N- protecteurs étant clivés après chaque couplage soit par hydrogénolyse, soit par hydrolyse en milieu acide fort et, le cas échéant, à déprotéger les groupes hydroxyle de l'aminoalcool ainsi obtenu par hydrolyse acide et à transformer si nécessaire le produit ainsi obtenu en l'un de ses sels pharmaceutiquement acceptables.

Lorsque l'acide aminé à introduire dans la séquence possède dans sa chaîne latérale une fonction susceptible de réagir, il convient de bloquer celle-ci par un groupe protecteur convenable que l'on élimine ultérieurement.

La protection de l'aminoacide N-terminal par le groupe R1 s'effectue selon des méthodes connues, soit avant le couplage du résidu

```
R1 NH-CH-CO
      |
      R2
```

avec l'aminoacide suivant, soit ultérieurement.

Lorsque le composé (I) porte un substituant R3 tel que le résidu correspondant est un aminoacide non naturel, cet aminoacide non naturel est préparé selon des méthodes connues. Il est ensuite couplé avec l'aminoacide suivant selon la méthode habituelle.

La partie aminoalcool est préparée par couplage d'un aminoalcool convenablement choisi avec l'aminoacide souhaité. Lorsque l'aminoalcool choisi n'est pas disponible commercialement, on peut le

préparer par réduction de l'ester d'aminoacide correspondant.

Les composés de la présente invention ont comme la pepstatine, une action inhibitrice marquée sur les protéases acides ; notamment ils ont une action inhibitrice sur l'activité rénine plasmatique humaine très importante et de façon générale, nettement supérieure à celle du produit naturel : la pepstatine. Des composés analogues qui ne possèdent pas de carbone tétra-ramifié sont décrits dans EP-A- 81.783.

Les produits selon l'invention ont été étudiés en ce qui concerne leurs propriétés thérapeutiques et notamment leur action inhibitrice enzymatique. Plus particulièrement, les composés ont été évalués "in vitro" sur l'inhibition de l'activité Rénine Plasmatique Humaine (A.R.P.).

I - METHODE D'EVALUATION

La méthode d'évaluation est inspirée de GUYENE (J. Clin. Endocrinol. Metab., 1976, 43, 1301). L'inhibition de l'A.R.P. est évaluée à partir d'un pool de plasmas humains, riche en rénine (15 à 20 mg d'angiotensine I libérée par millilitre et par heure), incubé pendant 60 minutes à 37° C, dans un tampon phosphate à pH 7,4 en présence de concentrations croissantes du produit à étudier.

Le plasma humain contient le substrat : l'angiotensinogène et l'enzyme : la rénine. L'angiotensine I libérée au cours de la réaction est mesurée par dosage radio-immunologique : Plasma Renin Activity Kit de Travenol (n° CA 533,553). Un inhibiteur de l'enzyme de conversion, le fluorure de phénylméthylsulfonyl (PMSF) est ajouté au milieu d'incubation. Le volume total d'incubation est de 555 microlitres répartis ainsi :

- 420 microlitres de plasma humain
- 11 à 50 microlitres du produit à étudier à des concentrations variables
- 119 à 80 microlitres de tampon phosphate
- 5 microlitres de PMSF.

On prépare une solution d'acide acétique IN dans le méthanol (19/1 en volume) et une solution de soude 1N dans le méthanol (2/1 en volume). Dans un mélange équivolumique de ces 2 solutions, on prépare une solution mère 0,001 M ,du produit à étudier. Les dilutions ultérieures sont alors effectuées dans le tampon phosphate. La quantité de solvant présente dans une solution n'interfère pas avec les résultats.

II - RESULTATS.

Les résultats sont exprimés par la dose de composé évaluée en moles qui inhibe de 50% (IC50) l'activité Rénine Plasmatique (A.R.P.) Humaine observée en l'absence d'inhibiteur.

Les résultats obtenus avec divers produits de l'invention sont représentés dans le tableau 1 suivant où figurent les IC50 de chaque molécule en ce qui concerne leur inhibition de l'Activité Rénine Plasmatique Humaine à pH 7,4. De 5 à 10 doses ont été nécessaires pour déterminer ces IC50. La pepstatine, en tant que substance de référence, est toujours testée en parallèle dans chaque expérience. Les résultats sont exprimés par leur valeur logarithmique (-Log IC50).

TABLEAU 1

| N° SR | Inhibition A.R.P. Humaine -Log IC 50 pH 7,4 |
|---|---|
| Pepstatine | 4,92 |
| 43 574 | 8,58 |
| 43 576 | 8,40 |
| 43 603 | 9,32 |
| 43 686 | 8,22 |
| 43 707 | 8,45 |
| 43 721 | 8,11 |
| 43 737 | 8,67 |
| 43 763 | 7,11 |
| 43 799 | 8,26 |
| 43 808 | a) |
| 43 842 | a) |
| 43 845 | a) |
| 43 860 | 7,48 |
| 43 925 | a) |
| | |
| 43 966 | 7,72 |
| 43 972 | b) 7,88 |
| 44 094 | 7,74 |
| 44 125 | 7,44 |

a) signifie que l'inhibition de l'ARP humaine est d'environ 90 % lorsque la concentration du produit est $10^{-10}$ molaire.

b) signifie que l'inhibition de l'ARP a été mesurée sur des plasmas de babouins.

On constate l'activité inhibitrice particulièrement élevée des produits selon l'invention.

La toxicité des produits selon l'invention est compatible avec leur utilisation en thérapeutique.

On peut donc envisager l'utilisation des produits selon l'invention dans les domaines thérapeutiques où l'inhibition des systèmes enzymatiques rénine-angiotensine est justifiée, notamment l'hypertension artérielle, l'ulcère gastroduodénal et les affections inflammatoires.

Ainsi, la présente invention a pour objet, selon un autre de ses aspects, des médicaments antihypertenseurs contenant les peptides de formule (I) ou leurs sels pharmaceutiquement acceptables en tant que

principes actifs.

Les aminoalcools peptidiques de la présente invention peuvent être utilisés en thérapeutique par voie injectable : intraveineuse, intramusculaire ou sous-cutanée. Ils sont utilisés dans un solvant tel que le sérum physiologique (solution saline isotonique) ou dans un tampon tel que le tampon phosphate ; ils peuvent également être mis en suspension dans un agent diluant, aqueux ou non-aqueux, pharmaceutiquement acceptable. Chaque unité de dosage peut contenir de 1 à 1000 mg de principe actif.

Les exemples suivants, non limitatifs, sont donnés à titre d'illustration de la présente invention.

Le pH des solutions dans un solvant organique est mesuré ou contrôlé en utilisant du papier pH indicateur humide.

Les points de fusion indiqués (Fc) sont pris à partir de solides triturés ; ils sont mesurés au tube capillaire.

Les indices de rotation (alpha D) sont mesurés à 25 ° C.

Pour l'enregistrement des spectres Infra Rouge (IR), le produit est mis en solution dans CH2Cl2 ou mélangé avec KBr pour former une pastille.

Enfin, les spectres de résonnance magnétique nucléaire (RMN) sont enregistrés à 250 MHz en solution dans le DMSO, l'étalon interne étant l'hexaméthyldisiloxane.

Les abréviations suivantes sont utilisées :

s : singulet
d : doublet
m : multiplet ou massif.

De plus,

. H ar signifie H aromatique ;
. H im signifie H imidazolique ;
. H pyr signifie H de la pyridine ;
. H (Sta réduite) signifie H de l'amino-4 méthyl-6 heptanediol 1,3 de configuration 3R,4S ou 3S,4S ;
. env. signifie environ.

Les déplacements chimiques (delta) sont mesurés en ppm.

EXEMPLE 1 :

```
                              CH2OH
                              |
Boc-Phe-Nle-Sta-Ile-NH-C-CH3
                              |
                              CH2OH
```

SR 43574

1 ° ) Boc-Nle-Sta-OCH3.

Dans un mélange équivolumique de CH2C12 et de dioxanne (16 ml) on fait réagir une solution de Boc-Nle-ONSu (900 mg), HOBt (421 mg), TFA-Sta-OCH3 (0,9 équivalent) contenant suffisamment de NMM pour amener le pH de la solution vers 7. Après 18 heures on évapore à siccité, ajoute de l'eau, extrait par de l'éther, lave la phase organique successivement par une solution de KHSO4, de l'eau, une solution de NaHCO3, puis une solution saline. Après séchage (MgSO4) et évaporation on isole une huile purifiée par chromatographie sur gel de silice (éluant pentane-acétate d'éthyle 1/1 en volume). Rendement 85%.
Fc = 79-81 ° C après trituration dans un mélange CH2Cl2-éther ;
alpha D = -53,1 (c = 1 ; MeOH).

## SPECTRE DE RMN

| Delta | Aspect | Protons | Attribution |
|---|---|---|---|
| 7,16 | d, J=9 Hz | 1 H | NHCO |
| 6,82 | d, J=8 Hz | 1 H | NHCO2 |
| 5,0 | d, J=4 Hz | 1 H | OH |
| 3,88-3,67 | m | 3 H | CH alpha (Nle) |
| | | | C<u>H</u>NH, C<u>H</u>OH |
| 3,50 | s | 3 H | OCH3 |
| 2,39-2,07 | m | 2 H | CH2CO (Sta) |
| 1,65-1,07 | m | 18 H | (CH3)3C, CH, CH2 |
| 0,84-0,71 | m | 9 H | CH3 (Nle et Sta) |

2°) Boc-Phe-Nle-Sta-OCH3.

On traite à 0°C pendant 10 minutes dans le TFA (8 ml) le peptide Boc-Nle-Sta-OCH3 (800 mg) puis évapore l'excès de solvant à TA sous le vide du rotavapor de Büchi. Au sel ainsi obtenu, on ajoute à 0°C une solution préparée dans CH2Cl2 (15 ml) de Boc-Phe-ONp (924 mg), HOBt (384 mg) et NMM (400 mg), puis amène le pH du milieu réactionnel vers 7 par addition de NMM supplémentaire. Après 24 heures, on évapore à siccité, ajoute de l'eau, extrait par AcOEt, lave la phase organique par une solution de KHSO4, plusieurs fois par une solution de Na2CO3, par de l'eau saline, puis sèche (MgSO4). Après évaporation du solvant et filtration sur gel de silice (éluant pentane-acétate d'éthyle 4/6 en volume), on isole le composé attendu sous forme d'une poudre blanche. Rendement 69%.
Fc = 126-129°C après recristallisation dans un mélange CH2Cl2-hexane ;
alpha D = -37,2 (c = 1 ; MeOH).

## SPECTRE DE RMN

| Delta | Aspect | Protons | Attribution |
|---|---|---|---|
| 7,83 | d, J=8 Hz | 1 H | NH CO |
| 7,49 | d, J=8 Hz | 1 H | NH CO |
| 7,27-7,04 | m | 5 H | C6H5 |
| 6,93 | d, J=8 Hz | 1 H | NH CO2 |
| 4,98 | d, J=4 Hz | 1 H | OH |
| | | | |
| | | | |
| 4,29-4,14 | m | 1 H | CH (alpha) |
| 4,14-4 | m | 1 H | CH (alpha) |
| 3,88-3,70 | m | 2 H | CH - NH - |
| | | | et CHOH (Sta) |
| 3,50 | s | 3 H | OCH3 |
| 2,95-2,56 | m | 2 H | CH2 C6H5 |
| 2,37-2,08 | m | 2 H | CH2CO (Sta) |
| 1,67-1,10 | m | 18 H | (CH3)3C ; |
| | | | CH2 (Nle) ; |
| | | | CH ; CH2 (Sta) |
| 0,86-0,70 | | 9 H | CH3 |

3°) Boc-Phe-Nle-Sta-OH.

Le solide précédent (750 mg) est hydrolysé à TA dans un mélange d'eau (5 ml) et de dioxanne (10 ml) par de la soude (1,3 équivalent) pendant 3 heures. On évapore à siccité au rotavapor de Büchi à TA, puis ajoute de l'eau, acidifie par une solution de KHSO4, extrait par 3 volumes d'AcOEt, lave par de l'eau salée, sèche (MgSO4). Après évaporation du solvant on isole un solide (rendement 95%) utilisé comme tel pour les réactions ultérieures. La RMN montre la disparition totale du pic à 3,50 ppm.
alpha D = -37,0 (c = 1 ; MeOH).

4°) (Boc-isoleucylamino)-2 méthyl-2 propanediol-1,3.

```
               CH2OH
               |
Boc-Ile-NH-C-CH3
               |
               CH2OH
```

On dissout 320 mg d'amino-2 méthyl-2 propanediol-1,3 dans 20 cm3 d'un mélange équivolumique DMF-CH2Cl2 ; on ajoute 1 g de Boc-Ile-ONsu et on laisse réagir 18 heures à TA sous agitation ; on ajoute à nouveau 320 mg d'amino-2 méthyl-2 propanediol-1,3 puis on laisse réagir 72 heures à TA sous agitation. Le mélange est évaporé à sec et le brut réactionnel est chromatographié sur silice en éluant par un mélange chloroforme/AcOEt à 1/9 en volume. On obtient 617 mg du produit attendu.
Fc = 123-126° C.

5°) SR 43574.

On traite 125 mg du composé obtenu précédemment à 0° C dans TFA pendant 15 minutes. Après évaporation de l'excès de solvant et lavage du sel à l'éther, on l'ajoute, en solution dans le minimum d'acétonitrile, à une solution de 200 mg du peptide Boc-Phe-Nle-Sta-OH dans 10 ml d'acétonitrile, en maintenant le pH voisin de 7-8 par addition de la quantité de NMM nécessaire. On ajoute ensuite 173 mg de Bop et laisse réagir pendant 24 heures. Après évaporation du solvant, le brut réactionnel est repris par de l'eau et extrait par AcOEt. On lave par une solution de NaHCO3, une solution de KHSO4 et une solution saline, on sèche puis évapore le solvant ; après séchage et évaporation du solvant, on cristallise le produit dans le mélange AcOEt/hexane, puis il est chromatographié sur silice et recristallisé dans le mélange CH2Cl2/éther isopropylique.
P = 171 mg ;
Fc = 107-110° C.

## SPECTRE DE RMN

| Delta | Aspect | Protons | Attribution |
|---|---|---|---|
| 8,0-7,48 | 3 d, J env. 8 Hz | 3 H | NHCO |
| 7,06-7,30 | m | 6 H | C6H5, NHCO (diol) |
| 6,96 | d, J env. 8 Hz | 1 H | NHCO2 |
| 4,86 | d, J env. 4 Hz | 1 H | OH (Sta) |
| 4,71 | t, J env. 4 Hz | 2 H | OH (diol) |
| 4,29-3,66 | m | 5 H | CH (alpha) ; CH (OH) |
| 3,50-3,25 | m | 4 H | (CH2)2 - OH |
| 2,97-2,57 | m | 2 H | CH2 C6H5 |
| 2,20-2,00 | m | 2 H | CH2CO |
| 1,70-1,00 | m | 21 H | C(CH3)3, CH2(Nle) CH, CH2(Sta) et |

| | | | | |
|---|---|---|---|---|
| | | | Ile | |
| 1,08 | s | 3 H | CH3 | |
| 0,91-0,68 | m | 15 H | CH3(Nle, Sta, Ile) | |

EXEMPLE 2 :

```
                                   CH2OH
                                   |
Boc-Phe-Nle-AHPPA-Ile-NH-C-CH2OH
                                   |
                                   CH2OH
```

SR 43576.

1°) Boc-Nle-AHPPA-OCH3.

Boc AHPPA OCH3 (3,0 g) est dissous dans TFA (20 ml) selon la technique habituelle, le sel obtenu est dissous dans CH2Cl2 puis neutralisé à 0° C par de la NMM. On ajoute alors au milieu réactionnel Boc-Nle-ONSu (3,3 g), HOBt (1,6 g) puis agite 18 heures à TA en maintenant le pH de la solution vers 7-8 par addition de NMM supplémentaire. Après les traitements habituels, le résidu est chromatographié sur gel de silice (éluant CH2Cl2-AcOEt, 1/1 en volume). Le peptide cristallise à l'évaporation du solvant au rotavapor de Büchi à froid (rendement 80%).
Fc = 89-90° C ;
IR (CH2Cl2) : 3420-1715-1675 cm-1.
alpha D = -61,5 (c = 1 ; MeOH).

2°) Boc-Phe-Nle-AHPPA-OCH3.

Le composé précédent (3,5 g) est dissous à 0° C dans TFA (30 ml). Le sel obtenu par évaporation de l'excès de réactif selon la méthode habituelle est dissous dans CH2Cl2 et neutralisé à 0° C par le NMM puis ajouté à 0° C à une solution préalablement obtenue de Boc-Phe-ONp (3,3 g), HOBt (1,33 g) et NMM (808 mg) dans CH2Cl2. On agite pendant 18 heures à TA puis lave la phase organique par de l'eau, par une solution de Na2CO3, par une solution de KHSO4, par de l'eau, puis sèche (MgSO4). Par évaporation du solvant, on isole un solide qui est trituré dans l'éther, puis filtré ; homogène en CCM. Rendement 75%.
Fc = 182-183° C.
alpha D = -50,7 (c = 1 ; MeOH).

SPECTRE DE RMN

| Delta | Aspect | Protons | Attribution |
|---|---|---|---|
| 7,27-7,04 | m | 10 H | ar |
| 5,23 | d, J=4 Hz | 1 H | OH |
| 4,27-3,80 | m | 4 H | CH (alpha) Phe et Nle, CHNH et CHOH |
| 3,49 | s | 3 H | OCH3 |
| 1,61-1,00 | m | 15 H | |
| 0,80 | t, J=6 Hz | 3 H | CH3 (Nle) |

3°) Boc-Phe-Nle-AHPPA-OH.

L'ester décrit à l'étape précédente (2 g) en solution dans le dioxanne (100 ml), est hydrolysé à TA pendant 4 heures par de la soude (178 mg) dissoute dans l'eau (10 ml). Le traitement ultérieur selon l'exemple 10, étape 2, fournit l'acide qui est trituré dans l'éther isopropylique, filtré et séché sous vide (rendement 95%).
Fc = 161-162°C.

4°) (Boc-isoleucylamino)-2 hydroxyméthyl-2 propanediol-1,3.

```
              CH2OH
              |
Boc-Ile-NH-C-CH2OH
              |
              CH2OH
```

A une solution de Boc-Ile-ONsu dans le DMF (10 ml), on ajoute du tris(hydroxyméthyl) aminométhane (1,32 g) en suspension dans 40 ml de DMF. Après 18 heures d'agitation, on concentre sous vide, reprend par de l'eau, extrait par AcOEt, sèche et concentre. On effectue une chromatographie sur silice en éluant par le mélange AcOEt/MeOH à 9/1 en volume. On recueille le produit attendu qui cristallise du mélange CH2Cl2/éther isopropylique.
P = 247 mg ;
Fc = 122-124°C ;
alpha D = -24,0 (c = 0,41 ; MeOH).

5°) SR 43576.

On débocule de façon habituelle 218 mg du composé obtenu ci-dessus par TFA. Le solide obtenu est repris par CH2Cl2, on ajoute 4 ml de DMF, la solution est neutralisée à froid par addition d'une quantité suffisante de DIPEA puis on ajoute le peptide Boc-Phe-Nle-AHPPA-OH (369 mg), du Bop (287 mg) et on laisse sous agitation pendant 18 heures à TA en maintenant le pH aux environs de 7. On concentre sous vide (pression : 5 mm de Hg), reprend par CH2Cl2, lave à l'eau, par une solution de Na2CO3, une solution de KHSO4, puis sèche et concentre. Le solide obtenu est chromatographié sur silice en éluant par le mélange chloroforme/méthanol à 95/5 en volume. Le produit attendu est recristallisé du mélange hexane-éther.
P = 265 mg ;
Fc = 94-95° C.

## SPECTRE DE RMN

| Delta | Aspect | Protons | Attribution |
|---|---|---|---|
| 7,96-7,65 | m | 3 H | NHCO |
| 7,29-7,00 | m | 11 H | C6H5 + NHCO |
| 6,95 | d, J=8 Hz | 1 H | NHCO2 |
| 5,07 | d, J env. 4 Hz | 1 H | OH |
| 4,63 | t, J env. 4 Hz | 3 H | CH2 - OH |
| 4,30-3,75 | m | 5 H | CH (alpha) ; CHOH |
| 3,57-3,38 | m | 6 H | (CH2)3 OH |
| 2,95-2,50 | m | 4 H | CH2C6H5 |
| 2,30-2,00 | m | 2 H | CH2CO |
| 1,68-0,93 | m | 18 H | C(CH3)3 ; CH2(Nle) CH, CH2 (Ile) |
| 0,86-0,66 | m | 9 H | CH3(Ile, Nle) |

EXEMPLE 3 :

```
                                                CH2OH
                                                |
(Pyridyl-3)-3 propionyl-Phe-Nle-AHPPA-Ile-NH-C-CH2OH
                                                |
                                                CH2OH
```

SR 43603.

On débocule de façon habituelle 240 mg du SR 43576, préparé à l'exemple précédent, par TFA. Après évaporation sous vide de l'excès de solvant, on obtient un résidu que l'on reprend par CH2Cl2, on ajoute 5 ml de DMF, neutralise à froid par addition d'une quantité suffisante de DIPEA, puis on ajoute 68 mg d'acide (pyridyl-3)-3 propionique et 84 mg de Bop. On laisse sous agitation 18 heures à TA en maintenant le pH aux environs de 7 par addition de DIPEA. On concentre sous vide (pression = 5 mm de Hg), reprend par AcOEt, lave à l'eau carbonatée, à l'eau, sèche et concentre. Après chromatographie sur silice en éluant par le mélange CHC13/MeOH à 9/1 en volume, on recueille le produit attendu qui concrétise du mélange hexane-éther.

P = 135 mg ;
Fc = 90-92 ° C.

## SPECTRE DE RMN

| Delta | Aspect | Protons | Attribution |
|---|---|---|---|
| 8,34-7,00 | m | 19 H | C5H4N ; C6H5 NHCO |
| 5,09 | d, J env. 4 Hz | 1 H | OH (AHPPA) |
| 4,64 | t, J env. 4 Hz | 3 H | OH (triol) |
| 4,57-3,70 | m | 5 H | CH (alpha) (Phe, Nle, Ile) CHOH et CHNH (AHPPA) |
| 3,57-3,38 | m | 6 H | CH2OH |
| 2,96-2,50 | m | 6 H | CH2-C5H4N CH2-C6H5 (Phe) CH2-C6H5 (AHPPA) |
| 2,34-2,00 | m | 4 H | CH2CO (AHPPA) C5H4NCH2-CH2CO |
| 1,68-0,92 | m | 9 H | CH2 (Nle) CH et CH2 (Ile) |
| 0,86-0,66 | m | 9 H | CH3 (Nle, Ile) |

EXEMPLE 4 :

```
                                              CH2OH
                                              |
(Pyridyl-3)-3 propionyl-Phe-His-Sta-Ile-NH-C-CH3
                                              |
                                              CH2OH
```

SR 43 737.

1 ° ) (Pyridyl-3)-3 propionyl-Phe-His-Sta-OCH3.

500 mg de Boc-Phe-His-Sta-OCH3 sont traités à 0 ° C pendant 5 minutes par 5 ml de TFA ; on évapore à siccité puis on précipite le sel dans l'éther. Celui-ci est repris par 15 ml d'acétonitrile puis on ajoute 132 mg d'acide (pyridyl-3)-3 propionique, 385 mg de Bop et on neutralise vers pH 7 par addition de DIPEA.

Après 24 heures, on évapore à siccité, ajoute de l'eau carbonatée et extrait par AcOEt. La phase organique est lavée plusieurs fois par une solution de Na2CO3, de l'eau, puis de l'eau saline, on sèche (MgSO4) puis évapore à siccité. Le résidu est dissous dans une solution 3M de KHSO4, on extrait les impuretés à l'éther. La phase aqueuse est neutralisée par une solution de NaHCO3. On extrait le précipité par plusieurs volumes d'AcOEt, la phase organique est lavée par une solution de Na2CO3, de l'eau saline puis séchée (MgSO4) et évaporée. On isole un solide blanc que l'on recristallise du mélange méthanol-éther isopropylique.
P = 97 mg ;
Fc = 168-170 °C ;
alpha D = -30,6 (c = 1 ; MeOH).

2°) (Boc-isoleucylamino)-2 méthyl-2 propanediol-1,3.

Ce composé est obtenu à l'exemple 1, étape 4.

3°) SR 43 737.

76,5 mg du composé obtenu à l'étape 1 sont dissous dans 6 ml de mélange méthanol/eau (5/1 en volume), on ajoute 5 gouttes de soude à 30% et laisse une nuit au réfrigérateur. On acidifie vers pH 3 par addition d'une solution de KHSO4 et évapore à siccité. Le résidu sec, en suspension dans 4 ml de DMF, est additionné en 1 fois, à 0 °C, à 5 ml de CH2Cl2 contenant 61 mg de Bop et le sel de TFA de 44 mg du composé obtenu à l'étape 2. On neutralise par addition de DIPEA et laisse sous agitation pendant 24 heures. On évapore à TA sous vide puis ajoute de l'eau carbonatée. Le précipité est extrait par CH2Cl2. On lave plusieurs fois par de l'eau saline. On sèche (Mg SO4) puis évapore le solvant. Le solide obtenu est chromatographié sur gel de silice en éluant par des mélanges croissants de méthanol dans le chloroforme.
Fc = 108-110 °C.

## SPECTRE DE RMN

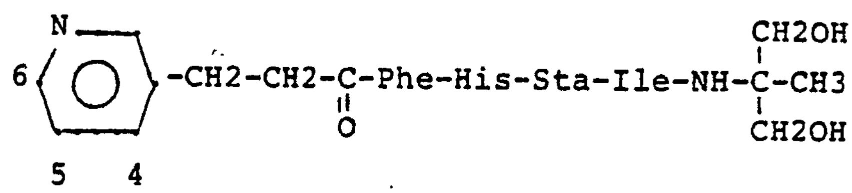

| Delta | Aspect | Protons | Attribution |
|---|---|---|---|
| 11,5-10,0 | s élargi | 1 H | NH imidazole (H1) |
| 8,39-8,27 | m | 3 H | H2, H6 (C5H4N) NHCO |
| 8,23 | d, J=8 Hz | 1 H | NHCO |
| 7,75 | d, J=8 Hz | 1 H | NHCO |
| 7,48 | s | 1 H | H2 (imidazole) |
| 7,42 | d, J env. 8 Hz | 1 H | H4 (C5H4N) |
| 7,27 | s | 1 H | NH - C - CH3 |
| 7,25-7,05 | m | 7 H | C6H5, H5 (C5H4N) NHCO |
| 6,79 | s | 1 H | H4 (imidazole) |
| 5,06-4,60 | m | 3 H | OH |
| 4,50-4,00 | m | 3 H | CH (alpha) |
| 3,81-3,64 | m | 2 H | CHNH et CHOH (Sta) |
| 3,50-3,00 | m | 4 H | CH2OH et H2O |
| 3,00-2,58 | m | 6 H | CH2 - C6H5<br>CH2 - imidazole<br>CH2 - C5H4N |
| 2,40-2,29 | m | 2 H | CH2 - CO |
| 2,19-1,95 | m | 2 H | CH2 - CO (Sta) |
| 1,08 | s | 3 H | CH3 - C - NH |
| 1,71-0,93 | m | 6 H | CH et CH2 (Sta et Ile) |
| 0,84-0,65 | m | 12 H | CH3 (Ile et Sta) |

EXEMPLE 5 :

```
                                                    CH2OH
                                                    |
(Pyridyl-3)-3 propionyl-Phe-Nle-Sta-Ile-NH-C-CH3
                                                    |
                                                    CH2OH
```

SR 43 707.

Ce composé est préparé à partir du SR 43 574 (138 mg) en opérant comme à l'exemple 3.
P = 60 mg ;
Fc = 86-87 °C.

## SPECTRE DE RMN

| Delta | Aspect | Protons | Attribution |
|---|---|---|---|
| 8,34-7,06 | m | 14 H | C5H4N, C6H5, NHCO et NH - C - CH3 |
| 4,80 | d, J env. 4 Hz | 1 H | OH (Sta) |
| 4,69 | t, J env. 4 Hz | 2 H | CH2 - OH |
| 4,58-4,00 | m | 3 H | CH (alpha) (Phe, Nle, Ile) |
| 3,84-3,67 | m | 2 H | CHOH et CHNH (Sta) |
| 3,50-3,25 | m | | CH2OH et pic H20 |
| 3,0-2,55 | m | 4 H | CH2C6H5, CH2C5H4N |
| 2,37-2,02 | m | 4 H | C5H4N-CH2CH2CO |

| | | | |
|---|---|---|---|
| | | | CH2CO (Sta) |
| 1,10 | s | 3 H | NH - C - CH3 |
| 1,71-0,92 | m | 12 H | CH et CH2 (Nle, Ile,Sta) |
| 0,92-0,68 | m | 15 H | CH3 (Ile, Nle, Sta) |

EXEMPLE 6 :

```
                               CH3
                               |
Boc-Phe-Nle-Sta-Ile-NH-C-CH2OH
                               |
                               CH3
```

SR 43 686.

```
                  CH3
                  |
1°) Boc-Ile-NH-C-CH2OH
                  |
                  CH3
```

A une solution de 1 g de Boc Ile ONSu dans 10 ml de CH2Cl2, on ajoute 0,534 g d'amino-2 méthyl-2 propanol dissous dans 5 ml de CH2Cl2. Après 20 minutes sous agitation, on essore l'insoluble puis on concentre.

2°) SR 43 686.

On débocule 302 mg du produit brut obtenu à l'étape précédente par 3 ml de TFA pendant 20 minutes à 2°C environ. Le résidu est dissous dans 10 ml de CH2Cl2, on ajuste la solution à pH 7 par addition de NMM puis on ajoute 535 mg de Boc-Phe-Nle-Sta OH et 442 mg de Bop. Après 48 heures sous agitation, on lave à l'eau saline, par une solution de KHSO4, une solution de NaHCO3 et de l'eau puis sèche et concentre. On chromatographie le résidu sur silice en éluant par l'acétate d'éthyle. Le produit attendu cristallise à froid dans l'éther.

## SPECTRE DE RMN

| Delta | Aspect | Protons | Attribution |
|---|---|---|---|
| 6,91-7,90 | m dont 4 d:<br>centrés à<br>6,94-7,5-<br>7,62-7,86<br>J = 8 Hz | 10 H | 5 H Ar, 5 NH |
| 3,70-4,90 | m dont un<br>d centré<br>à 4,85<br>J = 4 Hz | 7 H | OH (Sta),<br>OH ($CH_2OH$),<br>3 H alpha (Phe,<br>Nle, Ile)<br>2 H (Sta) |
| 2,02-3,38 | m | 8 H | $\underline{CH_2}$-OH, $\underline{CH_2}$-$C_6H_5$<br>$\underline{CH_2}$-NH, $\underline{CH_2}$-C(Sta)<br>‖<br>O |
| 0,69-2,69 | m dont 2 s:<br>à 1,23 et<br>1,11 | 36 H | $(CH_2)_3$ (Nle)<br>$CH_2$-CH (Sta)<br>Boc<br>$CH_3$<br>2 $CH_3$ de C-$CH_2OH$<br>$CH_3$<br>$CH_3$ (Nle, Sta,<br>Ile) |

EXEMPLE 7 :

```
                                  CH3
                                  |
Boc-Phe-(Pyr-3)Ala-Sta-Ile-NH-C-CH2OH
                                  |
                                  CH3
```

SR 43763.

1°) (R,S) Acétamido-2 éthoxycarbonyl-2 (pyridyl-3)-3 propionate d'éthyle.

A une solution de 11 g de sodium dans 300 ml de EtOH, on ajoute 48 g d'acétamidomalonate de diéthyle puis, en portant à reflux, on additionne 36 g de chlorhydrate de chlorométhyl-3 pyridine, on conserve 5 heures à reflux. On évapore EtOH, reprend par l'eau, extrait, sèche, concentre et chromatographie sur silice. Le mélange CH2Cl2-AcOEt, à 60/40 en volume, élue 23 g du composé attendu.

2°) (R,S)(Pyr-3)Ala, 2 HCl.

15 g du diester précédent sont portés à reflux dans 200 ml d'HCl 5 M pendant 5 heures. On concentre à sec, ajoute EtOH, évapore. Le procédé est réitéré jusqu'à recristallisation du dichlorhydrate : 15 g.

3°) (R,S) Boc(Pyr-3)Ala.

1 g du dichlorhydrate précédent est dissous dans un mélange à volume égal eau-dioxanne. On ajoute 350 mg de soude et 1 g de (Boc)20 puis on agite une nuit à TA. Le dioxanne est évaporé, on ajoute de l'eau, neutralise par 8 g d'Amberlite CG 120 L (forme acide), agite 1 heure à TA puis le milieu est mis sur une colonne contenant 8 g de la même résine. On élue par de l'eau distillée jusqu'à obtenir pH 5 puis élue par une solution d'ammoniaque à 4 % dans l'eau distillée. Les eaux sont concentrées. On recueille 0,69 g du produit attendu.

4°) Boc-(Pyr-3)Ala-Sta-OMe.

On agite pendant 3 jours à TA dans 10 ml d'acétonitrile un mélange de 465 mg de Sta-OMe, TFA, 500 mg de Boc-(Pyr-3)-Ala en présence de 780 mg de Bop, le milieu étant maintenu vers pH 7 par addition de DIPEA. On évapore alors à siccité, ajoute de l'eau, extrait par AcOEt, lave par une solution de Na2CO3, de l'eau, de l'eau saline, puis on sèche sur MgSO4 et évapore à siccité. Le résidu est chromatographié sur gel de silice par un mélange éluant CH2Cl2-AcOEt à 1/9 en volume qui élue successivement :

- l'isomère A le moins polaire : 150 mg ;
- l'isomère B le plus polaire : 180 mg.

```
                                      CH3
                                       |
5°) Boc-(Pyr-3)Ala-Sta-Ile-NH-C-CH2OH.
                                       |
                                      CH3
```

L'isomère le plus polaire obtenu à l'étape précédente (600 mg) est hydrolysé dans le mélange méthanol/dioxanne/eau (10/10/10 ; v/v/v) et l'on ajoute 70 mg de soude. Après 25 minutes, le produit de départ a disparu. On concentre à siccité, reprend par le dioxanne et ajoute de l'acétate d'éthyle chlorhydrique jusqu'à pH 4. On concentre à nouveau le milieu, solubilise dans 25 ml d'acétonitrile et ajoute DIPEA jusqu'à pH 7. On ajoute alors 500 mg de trifluoroacétate d'isoleucylamino-2 méthyl-2 propanediol-1,3 préalablement neutralisé dans l'acétonitrile par le DIPEA, puis on ajoute 650 mg de Bop et ajuste le pH entre 8 et 9 puis laisse une nuit sous agitation à température ambiante. Le milieu est concentré, repris par de l'eau carbonatée puis de l'acétate d'éthyle ; la phase organique est lavée sur sulfate acide de potassium, puis séchée et concentrée. Le brut est chromatographié sur silice, le mélange acétate d'éthyle/méthanol (95/5 ;v/v) élue le produit que l'on cristallise dans le mélange méthanol/éther. On recueille 420 mg.

6°) SR 43 763.

Cette étape est réalisée de la manière habituelle à partir de 390 mg du composé précédent et 250 mg de Boc-Phe-ONp. Après chromatographie sur silice en éluant par le mélange acétate d'éthyle/méthanol (90/10 ;v/v) on obtient 325 mg du produit attendu.

SR 43 763

| Delta | Aspect | Protons | Attribution |
|---|---|---|---|
| 8,48-7,05 | m | 13 H | H pyr, H ar, NHCO |
| | | | |
| 6,86 | d J=8 Hz | 1 H | NHCO2 |
| 4,91-4,65 | m | 3 H | OH (Sta), CH2-OH |
| 4,65-3,92 | m | 3 H | CH alpha (Phe,Ile |
| | | | (pyr-3) Ala) |
| 3,83-3,67 | m | 2 H | CHOH, CHNH (Sta) |
| 3,50-3,13 | m | - | CH2-OH et H2O |
| 3,10-2,50 | m | 4 H | CH2C6H5, CH2C5H4N |
| 2,16-1,90 | m | 2 H | CH2CO (Sta) |
| 1,70-1,00 | | ) | CH2 et CH (Sta) |
| 1,22 | s | ) 18 H | CH2 et CH (Ile) |
| 1,09 | s | ) | (CH3)3C, CH3 |
| 0,90-0,64 | m | 12 H | CH3 (Sta) |
| | | | CH3 (Ile) |

EXEMPLE 8 :

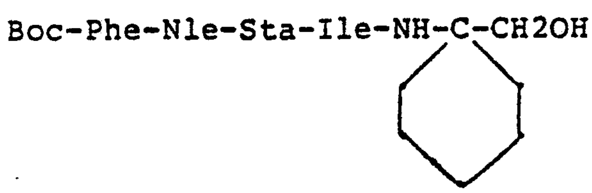

SR 43799.

1°) N-Boc-isoleucylamino-1 méthoxycarbonyl-1 cyclohexane.

On agite pendant 48 heures un mélange de Boc-Ile-OH (3,81 g), amino-1 méthoxycarbonyl-1 cyclohexane (2,6 g) et Bop (7,29 g) dans du chlorure de méthylène. On lave la phase organique à l'eau, au sulfate acide de potassium, au carbonate de sodium, sèche et concentre. On chromatographie sur silice en éluant par le mélange acétate d'éthyle/chlorure de méthylène (50/50 ,v/v) et on recueille le produit attendu qui cristallise à l'évaporation du solvant.

P = 4,4 g ;
Fc = 152-155°C ;
alpha D = -39° (c = 1 ; MeOH).

2°) N-Boc isoleucylamino-1 hydroxyméthyl-1 cyclohexane.

A une solution du produit précédent (1,5 g) dans l'éther, on ajoute par petites fractions 303 mg d'hydrure de sodium et d'aluminium et on agite 4 heures à TA. On verse le milieu réactionnel sur une solution glacée de sulfate acide de potassium, décante, sèche la phase éthérée et concentre sous vide. On obtient un solide blanc que l'on chromatographie sur silice. Le mélange acétate d'éthyle-hexane (50/50 ; v/v) élue le produit attendu qui cristallise à l'évaporation des solvants.
P = 1 g ;
Fc = 160-162°C ;
alpha D = -28,2 (c = 1 : MeOH).

3°) SR 43799.

Ce produit est obtenu par les méthodes de couplage habituelles.

**SPECTRE DE RMN**

| Delta | Aspect | Protons | Attribution |
|---|---|---|---|
| 7,85 | d J=8 Hz | 1 H | NHCO |
| 7,62 | d J=8 Hz | 1 H | NHCO |
| 7,50 | d J=8 Hz | 1 H | NHCO |
| 7,27-7,08 | m | 5 H | C6H5 |
| 7,08 | s | 1 H | NHCO |
| 6,93 | d J=8 Hz | 1 H | NHCO |
| 4,85 | d J=4 Hz | 1 H | OH |
| 4,53 | t J=6 Hz | 1 H | CH2-O<u>H</u> |
| 4,30-4,00 | m | 3 H | CH(alpha)(Phe, Nle,Ile) |
| 3,86-3,68 | m | 2 H | C<u>H</u>NH, C<u>H</u>OH(Sta) |
| 3,50-3,38 | m | 2 H | C<u>H2</u>OH |
| 2,96-2,55 | m | 2 H | C<u>H2</u>C6H5 |
| 2,18-0,64 | m | 48 H | (CH3)3, C<u>H2</u>CO(Sta) CH, CH2, CH3(Nle, Sta, Ile) CH2 (cyclohexane) |

EXEMPLE 9 :

```
                                                                  CH2OH
                                                                  |
(Pyridyl-3)-3 propionyl-Phe-(tauMe)His --ACHPA-Ile-NH-C-CH3
                                                                  |
                                                                  CH2OH
```

SR 43842.

1°) (tauMe)His-OMe, 2 HCl.

La (tauMe)His est préparée selon la méthode décrite dans J.R. Neth. Chem. Soc., 1978, 97, 293-295. 4,5 g du dichlorhydrate de ce produit sont dissous dans 50 ml de MeOH ; on sature par HCl gazeux à 10-15°C puis laisse sous agitation à TA pendant 72 heures. On concentre à sec, reprend 2 fois à l'éther puis le résidu est repris dans l'acétone où il cristallise ; après essorage, on obtient 4 g.

2°) Boc-Phe-(tauMe)His-OMe.

508 mg du produit précédent sont placés dans 25 ml de CH2Cl2 et amenés à pH 7 par addition de DIPEA. On ajoute 772 mg de Boc-Phe-ONp et 306 mg d'HOBt. Après 2 jours d'agitation à TA, on lave à l'eau glacée puis sèche et concentre. Par chromatographie sur silice et élution par un mélange AcOEt-MeOH à 9/1 en volume, on recueille 849 mg du produit attendu.

3°) Boc-Phe-(tauMe)His-ACHPA-OCH3.

On dissout à 10°C, dans 8 ml d'un mélange de méthanol et de dioxanne (1/3, vol/vol) 800 mg de Boc-Phe-(tauMe)His-OMe puis on ajoute 90 mg de soude dans 2 ml d'eau. On conserve à 10°C pendant 1 heure puis à TA pendant 1 heure 30. On acidifie par une solution de sulfate acide de potassium jusqu'à pH = 3 puis évapore à siccité. Le solide obtenu est mis en suspension dans 20 ml de DMF à 0°C en présence de 820 mg de Bop puis on ajoute, dans 7 ml de DMF, le sel TFA, ACHPA-OCH3 obtenu à partir de 620 mg de Boc-ACHPA-OCH3, on ajoute enfin 500 mg de DIPEA. On maintient à pH 6-7 et à une température comprise entre 0°C et 10°C pendant 2 heures puis ajoute DIPEA supplémentaire pour amener à pH 7 et laisse revenir à TA pendant une nuit. On évapore le solvant, ajoute de l'eau carbonatée, extrait 3 fois par de l'acétate d'éthyle, lave plusieurs fois par de l'eau carbonatée, par de l'eau, de l'eau saline, on sèche et évapore sous vide. Le solide est cristallisé du mélange méthanol-éther isopropylique. Puis on recristallise dans le minimum de méthanol à froid. On obtient 415 mg.
Fc = 164-166°C ;
alpha D = -34,3 (c = 0,61 ; MeOH).

4°) (Pyridyl-3)-3 propionyl-Phe-(tauMe)His-ACHPA-$OCH_3$.

On prépare le sel trifluoroacétique de 364 mg du produit obtenu à l'étape précédente. Ce sel est placé dans 10 ml d'acétonitrile, on ajoute 114 mg d'acide (pyridyl-3)-3 propionique, 336 mg de Bop et neutralise par addition de DIPEA, le milieu devient homogène puis un précipité apparaît. Le lendemain, le solide est filtré, lavé par de l'eau et de l'éther (solide 1). Aux jus de filtration, on additionne du carbonate de sodium aqueux, on évapore l'acétonitrile et extrait par de l'acétate d'éthyle. L'insoluble qui apparaît dans la phase organique est filtré ; le filtrat est dissous dans une solution de sulfate acide de potassium puis reprécipité par addition de bicarbonate de sodium (solide 2). Les solides 1 et 2 sont identiques et correspondent au produit attendu.
P = 215 mg ;
Fc = 211-215°C ;
alpha D = -36,7 (c = 0,27 ; MeOH).

5°) SR 43842.

Le composé précédemment obtenu (206 mg) est acidifié de la manière suivante. Il est mis en suspension dans un mélange d'eau (4 ml) et de méthanol (10 ml) puis on ajoute 5 gouttes de soude à 30 %, 4 ml de DMF et à nouveau 5 gouttes de soude à 30 %. Après 2 heures d'agitation, on observe une

dissolution presque totale. L'insoluble est filtré, on évapore le méthanol, acidifie vers pH 4-5 par une solution de sulfate acide de potassium et évapore à siccité. On traite à 0° C par 2 ml de TFA, 86 mg de

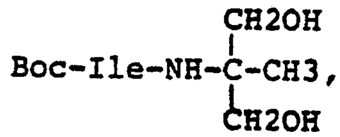

après évaporation, on dissout dans 3 ml de DMF et neutralise par la DIPEA. Dans 5 ml de DMF, on mélange le sel de TFA formé, l'acide précédent et 124 mg de Bop ; on refroidit à 0° C, additionne la quantité suffisante de DIPEA pour amener le pH vers 7-8 et laisse revenir à TA. Après 3 jours, on évapore le DMF, ajoute de l'eau carbonatée, extrait par 3 volumes d'eau carbonatée, de l'eau, de l'eau saline. Après évaporation on obtient un solide blanc (75 mg) qui recristallise par le mélange MeOH-AcOEt (27 mg). Fc = 119-124° C.

Les eaux mères de cristallisation sont chromatographiées sur silice.

## SPECTRE DE RMN

| Delta | Aspect | Protons | Attribution |
|---|---|---|---|
| 8,40-8,24 | m | 3 H | 2 H ortho pyr |
| | | | 1 NHCO |
| 8,17 | d J=8 Hz | 1 H | NHCO |
| 7,71 | d J=8 Hz | 1 H | NHCO |
| 7,45-7,07 | m | 10 H | 1 H (tauMe) |
| | | | 2 H pyr, 5 H ar, |
| | | | NHCO et NHC-CH3 |
| 6,82 | s | 1 H | 1 H (tauMe)His |
| 4,92 | large s | 1 H | OH (ACHPA) |
| 4,71 | t (J env. | 2 H | CH2-OH |
| | 4 Hz) | | |
| 4,50-3,62 | m | 5 H | CH(alpha)(Phe, |
| | | | (tauMe)His, Ile) |
| | | | CHNH, CHOH(ACHPA) |
| 3,50 | s | 3 H | CH3 (tauMe)His |
| 3,50-3,07 | m | 4 H | CH2OH(H2O) |
| 3,07-2,58 | m | 6 H | CH2-C5H4N |
| | | | CH2-C6H5 |
| | | | CH2-imidazole |
| 2,43-2,27 | m | 2 H | CH2-CO-Phe |
| 2,12-2,00 | m | 2 H | CH2CO(ACHPA) |
| 1,75-0,91 | m | 16 H | CH, CH2(ACHPA,Ile) |
| 1,08 | s | | CH3 (diol) |
| 0,91-0,56 | m | 6 H | CH3 (Ile) |

En utilisant les mêmes méthodes, les composés suivants (tableau 2) ont été préparés. Ils sont caractérisés par leur spectre de RMN.

TABLEAU 2

```
-------------------------------------------------------------------------
: SR 43 721                                                             :
:                                CH2OH                                  :
: Boc-Phe-Nle-ACHPA-Ile-NH-C-CH3                                        :
:                                CH2OH                                  :
: SR 43 808                                                             :
:                                                          CH2OH        :
: (Pyridyl-3)-3 propionyl-Phe-Nle-ACHPA-Ala-NH-C-CH3                    :
:                                                          CH2OH        :
: SR 43 845                                                             :
:                                                          CH2OH        :
: (Pyridyl-3)-3 propionyl-Phe-His-ACHPA-Ile-NH-C-CH3                    :
:                                                          CH2OH        :
: SR 43 860                                                             :
:                              CH3                                      :
: Boc-Phe-Nle-Sta-Ala-NH-C-CH2OH                                        :
:                              CH3                                      :
: SR 43 925                                                             :
:                                        CH2OH                          :
: Nicotinoyl-Phe-His-ACHPA-Ile-NH-C-CH3                                 :
:                                        CH2OH                          :
: SR 43 966                                                             :
:                                                                 CH3   :
: (Pyridyl-3)-3 propionyl-Phe-(tauMe)His-Sta-Ile-NH-C-CH2OH             :
:                                                                 CH3   :
: SR 43 972                                                             :
:                                                        CH2OH          :
: (Pyridyl-3)-3 propionyl-Phe-His-Sta-Ile-NH-C-CH3                      :
:                                                        CH2OH          :
: SR 44 094                                                             :
:                              CH2OH                                    :
: Boc-Phe-Nle-Sta-Ile-NH-C-CH2OH                                        :
:                              CH2OH                                    :
: SR 44 125                                                             :
:                                                          CH2OH        :
: Boc-Phe-(benzimidazolyl-2)Ala-ACHPA-Ile-NH-C-CH3                      :
:                                                          CH2OH        :
: SR 44 177                                                             :
:                                                          CH3          :
: (Pyridyl-3)-3 propionyl-Phe-His-ACHPA-Ile-NH-C-CH2OH                  :
:                                                          CH3          :
-------------------------------------------------------------------------
```

## SR 43 721

| Delta | Aspect | Protons | Attribution |
|---|---|---|---|
| 7,91 | d, J=8 Hz | 1 H | NHCO |
| 7,67 | d, J=8 Hz | 1 H | NHCO |
| 7,48 | d, J=8 Hz | 1 H | NHCO |
| | | | ' |
| 7,27-7,07 | m | 6 H | C6H5 ; NH-C-CH3 |
| | | | ' |
| 6,95 | d, J=8 Hz | 1 H | NHCO2 |
| 4,85 | d, J env. 4 Hz | 1 H | OH (ACHPA) |
| 4,68 | t, J env. 4 Hz | 2 H | CH2-OH |
| 4,31-4,0 | m | 3 H | CH (alpha) (Phe, Nle, Ile) |
| 3,90-3,68 | m | 2 H | CHOH et CHNH (ACHPA) |
| 3,50-3,19 | m | | CH2OH et pic H2O |
| 2,98-2,57 | m | 2 H | CH2C6H5 |
| 2,23-2,00 | m | 2 H | CH2-CO (ACHPA) |
| 1,23 | s | | C(CH3)3 |
| | | | ' |
| 1,08 | s | | CH3-C-NH |
| | | | ' |
| 1,77 | m | ) | CH et CH2 (Nle et Ile) |
| | | 31 H ) | |
| | | ) | CH et CH2 (ACHPA) |
| 0,66 | m | ) | CH3 (Nle et Ile) |

SR 43 808

| Delta | Aspect | Protons | Attribution |
|---|---|---|---|
| 7,1 -8,32 | m complexe dont 4 d centrés à 7,33, 7,42 8,07 J = 6 Hz | 14 H | 5 NHCO, 5 H ar, 4 H pyr |
| 3,24-4,83 | m complexe dont 1 d centré à 4,83 J = 6 Hz | 12 H | OH (ACHPA) 2 OH (diol) H alpha (Phe, Nle, Ala), CH-OH et CH-NH (ACHPA) 2 CH2-OH |
| 2,04-3 | m complexe dont 2 t centrés à 2,13 et 2,63 | 8 H | CH2-C6H5, CH2-pyr 2 CH2-CO |
| 0,49-1,75 | m complexe dont 1 s à 1,07 et 1 s à 0,81 | 27 H | 9 CH2 (Nle, ACHPA) 1 CH3 (Ala) 1 CH3 (diol) 1 CH3 (Nle) |

**SR 43 845**

| Delta | Aspect | Protons | Attribution |
|---|---|---|---|
| 8,40-8,78 | m | 3 H | 2 H ortho pyr |
| | | | 1 NHCO |
| 8,22 | d J=8 Hz | 1 H | NHCO |
| 7,66 | d J=8 Hz | 1 H | NHCO |
| 7,48 | s | 1 H | CH im |
| 7,42 | d J=8 Hz | 1 H | NHCO |
| 7,26 | s | 1 H | NH - C - |
| 7,23-7,07 | m | 7 H | 5 H ar |
| | | | 2 H pyr |
| 6,78 | large s | 1 H | CH im |
| 4,92 | large s | 1 H | OH (ACHPA) |
| 4,71 | t J env | 2 H | CH2-OH |
| | 4 Hz | | |
| 4,50-3,62 | m | 5 H | CH alpha (Phe, His |
| | | | Ile), CHOH et |
| | | | CHNH (ACHPA) |
| 3,50-3,07 | m | 4 H | CH2OH (H2O) |
| 3,07-2,58 | m | 6 H | CH2-C5H4N |
| | | | CH2-C6H5 |
| | | | CH2-imidazole |
| 2,43-2,27 | m | 2 H | CH2-CO-Phe |
| 2,12-2,00 | m | 2 H | CH2-CO (ACHPA) |
| 1,75-0,91 | m | 16 H | CH et CH2 (Ile, |
| | | | ACHPA) |
| 1,08 | s | 3 H | CH3 (diol) |
| 0,91-0,56 | m | 6 H | CH3 (Ile) |

alpha D = -35,5° (c = 0,21 ; méthanol) ;
Fc = 132-137° C (après concrétisation dans l'hexane).

## SR 43 860

| Delta | Aspect | Protons | Attribution |
|---|---|---|---|
| 7,90-6,86 | m | 10 H | 5 H ar, NH CO2, 3 NHCO, NH - C - |
| 4,82 | d J=4 Hz | 1 H | OH |
| 4,66 | m | 2 H | OH |
| 4,29-3,68 | m | 5 H | CH alpha (Phe, Nle: Ala), CHNH et CHOH (Sta) |
| 3,50-3,25 | m | | CH2OH et H2O |
| 2,97-2,56 | m | 2 H | CH2 (Phe) |
| 2,14-2,0 | m | 2 H | CH2CO (Sta) |
| 1,68-1,0 | m | 24 H | CH2 (Nle), CH et CH2 (Sta) CH3 à 1,08 (CH3)3C- à 1,25 |
| 0,89-0,71 | m | 9 H | CH3 (Nle et Sta) |

SR 43 925

| Deltà | Aspect | Protons | Attribution |
|---|---|---|---|
| 8,94-7,06 | m | 16 H | 4 H pyr, 5 H ar, |
| | | | 2 H im, 5 NHCO |
| 5,16-4,19 | m | 5 H | OH (partiellement |
| | | | échangés) |
| | | | CH alpha (Phe-His) |
| 4,09 | allure t | 1 H | CH alpha (Ile) |
| | J = 8 Hz | | |
| 3,83-3,66 | m | 2 H | CHNH, CHOH (ACHPA) |
| 3,50-2,79 | m | | CH2OH, CH2C6H5 |
| | | | H2O, CH2-imidazole |
| 2,20-1,93 | m | 2 H | CH2CO (ACHPA) |
| 1,73-0,88 | m | ) | CH, CH2 (ACHPA) |
| | | ) 19 H | CH, CH2 (Ile |
| 1,08 | s | ) | CH3 |
| 0,88-0,51 | m | 6 H | CH3 (Ile) |

**SR 43 966**

| Delta | Aspect | Protons | Attribution |
|---|---|---|---|
| 8,42-8,18 | m | 4 H | 2 H ortho pyr |
| | | | NHCO (His et Phe) |
| 7,65 | d J=8 Hz | 1 H | NHCO (Ile) |
| 7,46-7,04 | m | 10 H | 2 H pyr, H im, |
| | | | 5 H ar, NHCO (Sta) |
| | | | NH - C - |
| 6,81 | s | 1 H | H im |
| 4,97-4,70 | m | 2 H | OH (Sta), CH2-OH |
| 4,50-4,30 | m | 2 H | CH alpha (Phe His) |
| 4,05 | t J env | 1 H | CH alpha (Ile) |
| | 8 Hz | | |
| 3,77-3,61 | m | 2 H | CHNH et CHOH (Sta) |
| 3,43-3,19 | m | | CH2OH et H2O |
| 3,50 | s | 3 H | N-CH3 |
| 3,02-2,57 | m | 6 H | CH2 (Phe, His, |
| | | | C5H4N) |
| 2,41-2,27 | m | 2 H | CH2CO |
| 2,14-1,97 | m | 2 H | CH2CO (Sta) |
| 1,70-0,90 | m | 12 H | CH et CH2 (Ile) |
| | | | CH et CH3 (Sta) |
| | | | CH3 NH-C(CH3)2 |
| | | | CH2-OH |
| 0,90-0,61 | m | 12 H | CH3 (Ile) |
| | | | CH3 (Ala) |

SR 43 972

| Delta | Aspect | Protons | Attribution |
|---|---|---|---|
| 11,80 | large s | 1 H | NH im |
| 8,44-8,17 | m | 4 H | 2 H ortho pyr |
| | | | 2 H NHCO |
| 7,70 | d J=8 Hz | 1 H | NHCO |
| 7,50-7,03 | m | 10 H | 2 NHCO + H im |
| | | | 5 H ar, 2 H pyr |
| 6,79 | s | 1 H | H im |
| 4,95-3,62 | m | 8 H | 3 OH, CH alpha |
| | | | (Phe,His,Ile) |
| | | | CHNH et CHOH (Sta) |
| 3,50-3,14 | m | | CH2OH (et H20) |
| 3,06-1,91 | m | 10 H | CH2 (His, Phe) |
| | | | CH2 (C5H4N) |
| | | | CH2CO (Sta) |
| | | | CH2-CH2-CO |
| 1,71-0,91 | m | 8 H | CH et CH2 (Sta et |
| | | | Ile) |
| | | | CH2-CH2-CH2-CO |
| 1,08 | s | 3 H | CH3 |
| 0,91-0,55 | m | 12 H | CH3 (Ile et Sta) |

## SR 44 094

| Delta | Aspect | Protons | Attribution |
|---|---|---|---|
| 7,90-6,87 | m | 10 H | 5 H ar, 1 NHCO2, 3 NHCO, NH - C - |
| 4,83 | d J=4 Hz | 1 H | OH |
| 4,62 | m | 3 H | OH |
| 4,31-3,41 | m | 11 H | CH alpha (Phe, Nle: Ile), CH et CHOH (Sta), CH2OH |
| 2,98-2,57 | m | 2 H | CH2C6H5 |
| 2,29-2,00 | m | 2 H | CH2CO (Sta) |
| 1,70-0,63 | m | autres | CH, CH2 et CH3 aliphatiques |

## SR 44 125

| Delta | Aspect | Protons | Attribution |
|---|---|---|---|
| 12,2 | large s | 1 H | NH benzimidazole |
| 8,48-6,96 | m | 14 H | 5 H ar, C6H4 |
| | | | (benzimidazole) |
| | | | NHCO2, NHCO, NH-C- |
| 5,26 | d J=5 Hz | 1 H | OH (ACHPA) |
| 4,78-4,61 | m | 3 H | CH2-OH, CH alpha |
| | | | benzimidazole |
| 4,23-3,67 | m | 4 H | CH alpha (Phe Ile) |
| | | | CHNH et |
| | | | CHOH (ACHPA) |
| 3,50-2,59 | m | | CH2OH, CH2 (Phe) |
| | | | CH2(benzimidazole) |
| 2,27-2,08 | m | 2 H | CH2CO (ACHPA) |
| 1,75-0,55 | m | 34 H | CH et CH2 (ACHPA) |
| | | | CH, CH2, CH3 (Ile) |
| | | | -C(CH3)3, -CH3 |

**SR 44 177**

| Delta | Aspect | Protons | Attribution |
|---|---|---|---|
| 11,81 | large s | 1 H | NH imidazole |
| 8,42-7,05 | m | 13 H | NHCO, NH - C - |
| | | | 4 H pyr, H im |
| | | | 5 H ar |
| 6,80 | large s | 1 H | CH im |
| 5,0-5,49 | large m | 2 H | OH |
| 4,5-4,34 | m | 2 H | CH alpha (Phe et |
| | | | His) |
| 4,05 | t J=8 Hz | 1 H | CH alpha (Ile) |
| 3,82-3,65 | m | 2 H | CHNH et |
| | | | CHOH (ACHPA) |
| 3,40-3,20 | m | 2 H | CH2OH (H2O) |
| 3,05-2,27 | m | 8 H | CH2-C6H5 |
| | | | CH2-imidazole |
| | | | CH2-pyridine |
| | | | CH2CO (Phe) |
| 2,17-1,97 | m | 2 H | CH2CO (ACHPA) |
| 1,74-0,94 | m | 22 H | CH et CH2 (ACHPA, |
| | | | Ile), CH3 |
| 0,91-0,55 | m | 6 H | CH3 (Ile) |

Fc = 125-128° C (trituré dans l'éther)
alpha D = -32,3° (c = 0,6 : méthanol).

**Revendications**

**Revendications pour les Etats Contractants: BE, CH, DE, FR, GB, IT, LI, LU, NL, SE**

**1.** Dérivé aminoalcool peptidique de formule :

```
R1-NH-CH-CO-NH-CH-CO-NH-CH-CHOH-CH2-CO-X-N-R5      (I)
      |         |         |                |
      R2        R3        CH2              R4
                          |
                          Q
```

dans laquelle :

- $R_1$ représente un groupe acyle choisi parmi les groupes alkylcarbonyle, alcoxycarbonyle, arylcar-

bonyle, arylalkylcarbonyle, arylalcoxycarbonyle, hétérocyclylcarbonyle, hétérocyclylalkylcarbonyle, dans lequel le groupe alkyle contient de 1 à 10 atomes de carbone et est éventuellement substitué par un groupe hydroxy, hétérocyclylcarbonylalkylcarbonyle, hétérocyclylalcénylcarbonyle, cycloalkylcarbonyle, ou un groupe (alkyle contenant jusqu'à 6 atomes de carbone)-sulfonyle non substitué ou substitué sur l'alkyle par un groupe amino libre ou portant un groupe protecteur ou par un phényle ; ou un groupe phénylsulfonyle non substitué ou substitué sur le noyau phényle par un alkyle contenant jusqu'à 6 atomes de carbone ;

- $R_2$ représente un groupe alkyle contenant jusqu'à 6 atomes de carbone non substitué ou substitué par un phényle, naphtyle, cyclohexyle, ou pyridyle ou $R_2$ représente un radical phényle, naphtyle, cyclohexyle ou pyridyle ;
- $R_3$ représente l'hydrogène, un alcényle contenant jusqu'à 6 atomes de carbone, un phényle, un naphtyle, un cycloalkyle contenant 3 à 6 atomes de carbone, un groupe hétérocyclique monocyclique à 5 ou 6 chaînons non substitué ou substitué avec un alkyle contenant jusqu'à 6 atomes de carbone ou trifluorométhyle ; ou bien un alkyle contenant jusqu'à 6 atomes de carbone non substitué ou substitué par un groupe amino libre ou portant un groupe protecteur, par un groupe di(alkyle contenant jusqu'à 6 atomes de carbone)amino, par un carboxyle libre ou estérifié avec un alkyle contenant jusqu'à 6 atomes de carbone ou un benzyle, par un carbamoyle libre ou substitué avec un ou deux alkyles contenant jusqu'à 6 atomes de carbone ou avec un phényle, par un groupe hydroxy, alcoxy contenant jusqu'à 6 atomes de carbone ou benzyloxy, par un groupe pyridylméthyloxy, par un groupe alkylthio contenant jusqu'à 6 atomes de carbone, (alkyle contenant jusqu'à 6 atomes de carbone)sulfinyle ou (alkyle contenant jusqu'à 6 atomes de carbone)sulfonyle, par un phényle non substitué ou substitué par un hydroxyle, par un naphtyle, par un cycloalkyle contenant de 3 à 6 atomes de carbone, par un groupe hétérocyclique monocyclique à 5 ou 6 chaînons non substitué ou substitué avec un alkyle contenant jusqu'à 6 atomes de carbone ou un trifluorométhyle ou par un groupe hétérocyclique bicyclique non substitué ou substitué avec un alkyle contenant jusqu'à 6 atomes de carbone ou un trifluorométhyle ;
- $R_4$ représente l'hydrogène ou un alkyle contenant jusqu'à 6 atomes de carbone non substitué ou substitué par un radicale indolyle, pyridyle, imidazolyle ou phényle ;
- $R_5$ représente un groupe hydrocarboné aliphatique ou cyclique de 3 à 20 atomes de carbone non substitué ou substitué par un groupe phényle, hydroxyphényle ou amino, ledit groupe hydrocarboné contenant au moins un groupe hydroxyle et au moins un atome de carbone ne présentant pas de liaison C-H ;
- Q représente l'isopropyle, le phényle ou le cyclohexyle formant respectivement avec le radical

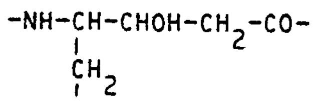

le résidu de l'aminoacide Sta(acide amino-4 hydroxy 3-méthyl-6 heptanoïque ou statine) ; AHPPA (acide amino-4 hydroxy-3 phényl-5 pentanoïque) ou ACHPA (acide amino-4 cyclohexyl-5 hydroxy-3 pentanoïque) ;
- X est soit une liaison directe, soit le résidu d'un aminoacide tel Ala (alanine), Nva (norvaline), Val (valine), Leu (leucine), Nle (norleucine), Ile (isoleucine), Phg (phénylglycine), Abu (acide $\alpha$-aminobutyrique), Cpg (cyclopentylglycine) ;

ainsi que les sels éventuels pharmaceutiquement acceptables dudit aminoalcool peptidique avec les acides minéraux ou organiques ou avec des métaux alcalins ou alcalino-terreux.

2. Dérivé aminoalcool peptidique selon la revendication 1, de formule :

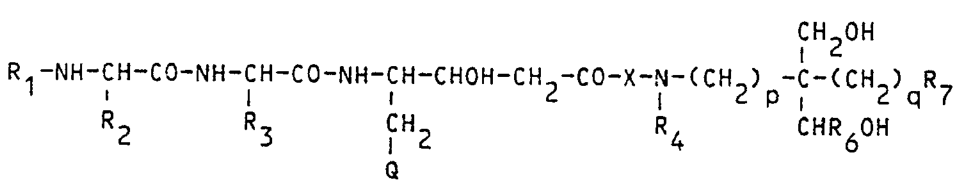

dans laquelle $R_1$, $R_2$, $R_3$, Q, X et $R_4$ sont tels que définis dans la revendication 1,

- p représente un nombre entier compris entre 0 et 5 ;
- q représente un nombre entier compris entre 0 et 5 ;
- $R_6$ représente l'hydrogène ou un alkyle contenant jusqu'à 6 atomes de carbone et
- $R_7$ représente l'hydrogène, un groupement hydroxyle, un groupement amino, un groupement carboxy, un groupement pyridyle ou un groupement phényle non substitué ou substitué par un hydroxyle, à la condition que, lorsque q = 0, $R_7$ soit différent de l'hydrogène.

**3.** Dérivé aminoalcool peptidique selon la revendication 1, de formule :

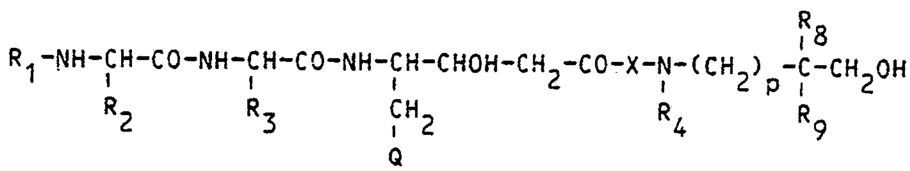

dans laquelle $R_1$, $R_2$, $R_3$, Q, X et $R_4$ sont tels que définis dans la revendication 1,

- p représente un nombre entier compris entre 0 et 5 ;
- $R_8$ représente un alkyle contenant jusqu'à 6 atomes de carbone ;
- $R_9$ représente un alkyle contenant jusqu'à 6 atomes de carbone non substitué ou substitué par un groupe amino libre ou portant un groupe protecteur ;
- ou $R_8$ et $R_9$ ensemble forment avec l'atome de carbone auquel ils sont reliés un cycloalkyle de 3 à 8 atomes de carbone.

**4.** Dérivé aminoalcool peptidique selon l'une quelconque des revendications 1 à 3, caractérisé en ce que $R_1$ représente l'un des groupements suivants :

$$(CH_3)_3C-O-\underset{\underset{O}{\Vert}}{C}-$$

$$(CH_3)_2CH-CH_2-\underset{\underset{O}{\Vert}}{C}-$$

$$C_6H_5-(CH_2)_a-\underset{\underset{O}{\Vert}}{C}-\text{, où } a = 0\text{, 1 ou 2}$$

$$(C_6H_5-CH_2)_2CH-\underset{\underset{O}{\Vert}}{C}-$$

$$C_6H_5-CH_2-O-\underset{\underset{O}{\Vert}}{C}-$$

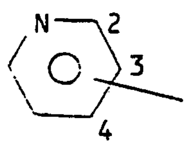

$$(CH_2)_b-\underset{\underset{O}{\Vert}}{C}-\text{, où } b = 0\text{, 1, 2, 3, 4, 5 ou 6}$$

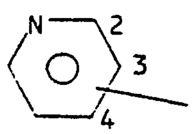

$$D-(CH_2)_n-\underset{\underset{O}{\Vert}}{C}-\text{, où } D = -\underset{\underset{OH}{|}}{\overset{*}{C}}H- \text{ ou } -\underset{\underset{O}{\Vert}}{C}-$$

et n = 1, 2, 3, 4 ou 5

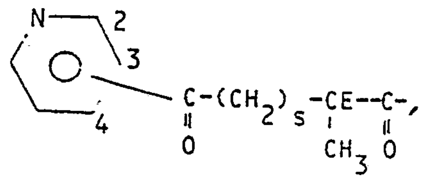 , où E = -H ou $-CH_3$ et s = 1, 2, 3 ou 4

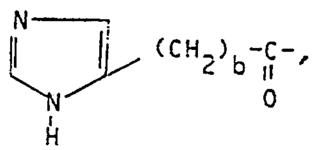 , où b = 0, 1, 2, 3, 4, 5 ou 6

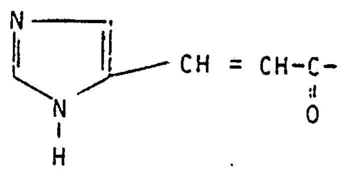

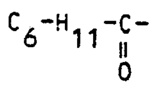

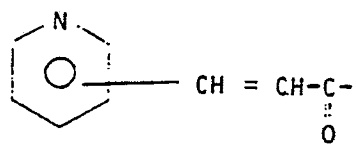

$NH_2CH_2CH_2-SO_2-$

$BocNHCH_2CH_2-SO_2-$

$Z-NHCH_2CH_2-SO_2-$

$A-SO_2-$, où A est un alkyle contenant jusqu'à 6 atomes de carbone ;

$C_6H_5-(CH_2)_a-SO_2$, où a = 0, 1 ou 1
ou un des sels éventuels d'acides minéraux ou organiques pharmaceutiquement acceptables desdits aminoalcools peptidiques.

5. Derivé aminoalcool peptidique selon l'une quelconque des revendications 1 à 3, caractérisé en ce que $R_1$ représente un groupe choisi parmi les groupes suivants :
- isovaléryle
- (pyridyl-2)-4 oxo-4 butyryle
- (pyridyl-2)-4 hydroxy-4 butyryle
- (pyridyl-3)-3 propionyle
- (pyridyl-3)-4 butyryle
- nicotinoyle

- benzènesulfonyle.

**6.** Dérivé aminoalcool peptidique selon l'une quelconque des revendications 1 à 5, caractérisé en ce que $R_5$ contient de 1 à 3 groupes hydroxyle.

**7.** Dérivé aminoalcool peptidique selon l'une quelconque des revendications 1 à 6, caractérisé en ce que $R_3$ est tel que le résidu -NH-CH($R_3$)CO- représente le résidu (tauMe)His.

**8.** Dérivé aminoalcool peptidique de formule :

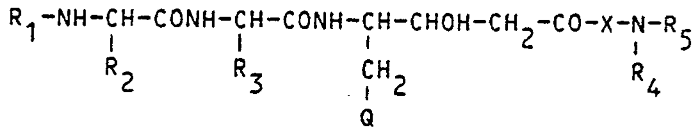

(I)

dans laquelle :

- $R_1$ représente un groupe choisi parmi les groupes suivants : isovaléryle, tert-butyloxycarbonyle, (pyridyl-3)-3 propionyle, (pyridyl-3)-4 butyryle, nicotinoyle ;
- $R_2$ représente un benzyle ;
- $R_3$ représente n-butyle ou un méthyle substitué par un radical hétérocyclique choisi parmi imidazolyl-4, méthyl-1 imidazolyl-4, méthyl-2- imidazolyl-4, pyridyl-3, benzimidazolyl-2 ;
- $R_4$ est l'hydrogène ;
- Q représente l'isopropyle, le phényle ou le cyclohexyle formant respectivement avec le radical

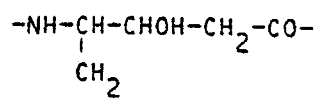

le résidu de l'aminoacide Sta(acide amino-4 hydroxy-3 méthyl-6 heptanoïque ou statine) ; AHPPA (acide amino-4- hydroxy-3 phényl-5 pentanoïque) ou ACHPA (acide amino-4 cyclohexyl-5 hydroxy-3 pentanoïque) ;
- X représente le résidu de l'un des aminoacides Ile (isoleucine) ou Ala (alanine) ;
- $R_5$ représente un groupe hydrocarboné aliphatique ou cyclique de 3 à 20 atomes de carbone non substitué ou substitué par un groupe phényle, hydroxyphényle, amino, ledit groupe hydrocarboné contenant simultanément au moins un groupe hydroxyle et au moins un atome de carbone ne présentant pas de liaison C-H ;

ainsi que les sels éventuels pharmaceutiquement acceptables dudit aminoalcool peptidique avec les acides minéraux ou organiques ou avec des métaux alcalins ou alcalino-terreux.

**9.** Dérivé aminoalcool peptidique selon la revendication 8, dans lequel $R_5$ représente un groupement choisi parmi

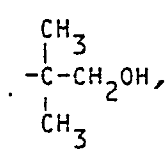
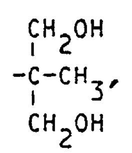
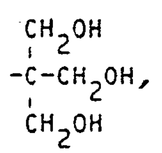
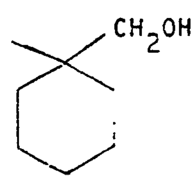

**10.** Procédé pour la préparation des dérivés aminoalcools peptidiques selon la revendication 1, caractérisé en ce que l'on traite un aminoalcool de formule :

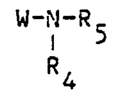

dans laquelle W est un groupe protecteur ou l'hydrogène et $R_4$, $R_5$ sont tels que définis dans la revendication 1 et dont les groupes hydroxyle sont éventuellement protégés, avec l'ester d'alkyle (contenant jusqu'à 6 atomes de carbone) de l'acide aminé de formule :

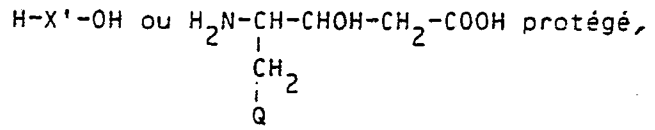

dans laquelle X' a la définition donnée dans la revendication 1 pour X, mais X' est autre qu'une liaison directe et Q est tel que défini ci-dessus, puis on procède, étape par étape, à l'élongation de la chaîne peptidique par couplage avec les acides aminés ou les fragments suivants, convenablement protégés, les différentes opérations étant effectuées en utilisant soit un ester activé de l'aminoacide ou du fragment à coupler, soit l'aminoacide N- protégé en présence de DCCI, les groupes N- protecteurs étant clivés après chaque couplage soit par hydrogénolyse, soit par hydrolyse en milieu acide fort et, le cas échéant, on déprotège les groupes hydroxyle de l'aminoalcool ainsi obtenu par hydrolyse acide ; et l'on transforme, si nécessaire, le produit ainsi obtenu dans un de ses sels d'acides minéraux ou organiques ou des métaux alcalins ou alcalino-terreux.

**11.** Médicaments caractérisés en ce qu'ils contiennent un produit selon la revendication 1 en tant qu'ingrédient actif.

**12.** Médicaments selon la revendication 11 utilisables notamment pour le traitement de la tension artérielle, caractérisés en ce qu'ils contiennent de 1 à 1 000 mg de produit selon la revendication 1 par unité de dosage en mélange avec un excipient pharmaceutique.

**Revendications pour l'Etat contractant suivant: AT**

**1.** Procédé pour l'obtention de dérivés aminoalcools peptidiques de formule :

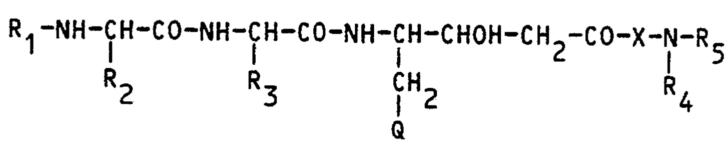

(I)

dans laquelle :

- $R_1$ représente un groupe acyle choisi parmi les groupes alkylcarbonyle, alcoxycarbonyle, arylcarbonyle, arylalkylcarbonyle, arylalcoxycarbonyle, hétérocyclylcarbonyle, hétérocyclylalkylcarbonyle dans lequel le groupe alkyle contient de 1 à 10 atomes de carbone et est éventuellement substitué par un groupe hydroxy, hétérocyclylcarbonylalkylcarbonyle, hétérocyclylalcénylcarbonyle, cycloalkylcarbonyle, ou un groupe (alkyle contenant jusqu'à 6 atomes de carbone)-sulfonyle non substitué ou substitué sur l'alkyle par un groupe amino libre ou portant un groupe protecteur ou par un phényle ; ou un groupe phénylsulfonyle non substitué ou substitué sur le noyau phényle par un alkyle contenant jusqu'à 6 atomes de carbone ;
- $R_2$ représente un groupe alkyle contenant jusqu'à 6 atomes de carbone non substitué ou substitué par un phényle, naphtyle, cyclohexyle, ou pyridyle ou $R_2$ représente un radical phényle, naphtyle, cyclohexyle ou pyridyle ;
- $R_3$ représente l'hydrogène, un alcényle contenant jusqu'à 6 atomes de carbone, un phényle, un naphtyle, un cycloalkyle contenant 3 à 6 atomes de carbone, un groupe hétérocyclique monocyclique à 5 ou 6 chaînons non substitué ou substitué avec un alkyle contenant jusqu'à 6 atomes

de carbone ou trifluorométhyle, ou bien un alkyle contenant jusqu'à 6 atomes de carbone non substitué ou substitué par un groupe amino libre ou portant un groupe protecteur, par un groupe di(alkyle contenant jusqu'à 6 atomes de carbone)amino, par un carboxyle libre ou estérifié avec un alkyle contenant jusqu'à 6 atomes de carbone ou un benzyle, par un carbamoyle libre ou substitué avec un ou deux alkyles contenant jusqu'à 6 atomes de carbone ou avec un phényle, par un groupe hydroxy, alcoxy contenant jusqu'à 6 atomes de carbone ou benzyloxy, par un groupe pyridylméthyloxy, par un groupe alkylthio contenant jusqu'à 6 atomes de carbone, (alkyle contenant jusqu'à 6 atomes de carbone)sulfinyle ou (alkyle contenant jusqu'à 6 atomes de carbone)sulfonyle, par un phényle non substitué ou substitué par un hydroxyle, par un naphtyle, par un cycloalkyle contenant de 3 à 6 atomes de carbone, par un groupe hétérocyclique monocyclique à 5 ou 6 chaînons non substitué ou substitué avec un alkyle contenant jusqu'à 6 atomes de carbone ou un trifluorométhyle ou par un groupe hétérocyclique bicyclique non substitué ou substitué avec un alkyle contenant jusqu'à 6 atomes de carbone ou un trifluorométhyle ;

- $R_4$ représente l'hydrogène ou un alkyle contenant jusqu'à 6 atomes de carbone non substitué ou substitué par un radical indolyle, pyridyle, imidazolyle ou phényle ;
- $R_5$ représente un groupe hydrocarboné aliphatique ou cyclique de 3 à 20 atomes de carbone non substitué ou substitué par un groupe phényle, hydroxyphényle ou amino, ledit groupe hydrocarboné contenant au moins un groupe hydroxyle et au moins un atome de carbone ne présentant pas de liaison C-H ;
- Q représente l'isopropyle, le phényle ou le cyclohexyle formant respectivement avec le radical

$$-NH-\underset{\substack{|\\CH_2\\|}}{CH}-CHOH-CH_2-CO-$$

le résidu de l'aminoacide Sta(acide amino-4 hydroxy-3 méthyl-6 heptanoïque ou statine) ; AHPPA (acide amino-4 hydroxy-3 phényl-5 pentanoïque) ou ACHPA (acide amino-4 cyclohexyl-5 hydroxy-3 pentanoïque) ;

- X est soit une liaison directe, soit le résidu d'un aminoacide tel que Ala (alanine), Nva (norvaline), Val (valine), Leu (leucine), Nle (norleucine), Ile (isoleucine), Phg (phénylglycine), Abu (acide $\alpha$-aminobutyrique), Cpg (cyclopentylglycine).

ainsi que les sels éventuels pharmaceutiquement acceptables dudit aminoalcool peptidique avec les acides minéraux ou organiques ou avec des métaux alcalins ou alcalino-terreux, caractérisé en ce qu'il consiste à traiter un aminoalcool de formule :

$$W-\underset{\substack{|\\R_4}}{N}-R_5$$

dans laquelle W est un groupe protecteur ou l'hydrogène et $R_4$, $R_5$ sont tels que définis ci-dessus et dont les groupes hydroxyle sont éventuellement protégés, avec l'ester d'alkyle (contenant jusqu'à 6 atomes de carbone) de l'acide aminé de formule :

$$H-X'-OH \text{ ou } H_2N-\underset{\substack{|\\CH_2\\|\\Q}}{CH}-CHOH-CH_2-COOH \text{ protégé ,}$$

dans laquelle X' a la définition donnée ci-dessus pour X, mais X' est autre qu'une liaison directe et Q est tel que défini ci-dessus, puis on procède, étape par étape, à l'élongation de la chaîne peptidique par couplage avec les acides aminés ou les fragments suivants, convenablement protégés, les différentes opérations étant effectuées en utilisant soit un ester activé de l'aminoacide ou du fragment à coupler, soit l'aminoacide N- protégé en présence de DCCI, les groupes N- protecteurs étant clivés

après chaque couplage, à déprotéger le cas échéant les groupes hydroxyle de l'aminoalcool ainsi obtenu et éventuellement à transformer ledit produit en l'un de ses sels pharmaceutiquement acceptabes.

**2.** Procédé selon la revendication 1, caractérisé en ce que l'on utilise un aminoalcool de formule :

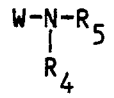

dans laquelle $R_4$ et W sont tels que définis dans la revendication 1 et $R_5$ répond à la formule :

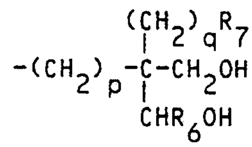

dans laquelle :

- p représente un nombre entier compris entre 0 et 5 ;
- q représente un nombre entier compris entre 0 et 5 ;
- $R_6$ représente l'hydrogène ou un alkyle contenant jusqu'à 6 atomes de carbone ;
- $R_7$ représente l'hydrogène, un groupement hydroxyle, un groupement amino, un groupement carboxy, un groupement pyridyle ou un groupement phényle non substitué ou substitué par un hydroxyle, à la condition que, lorsque q = 0, $R_7$ est différent de l'hydrogène.

**3.** Procédé selon la revendication 1, caractérisé en ce que l'on utilise un aminoalcool de formule :

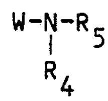

dans laquelle $R_4$ et W sont tels que définis dans la revendication 1 et $R_5$ est le groupe de formule :

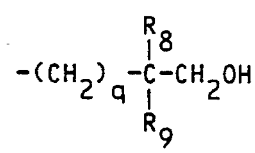

dans laquelle

- p représente un nombre entier compris entre 0 et 5 ;
- $R_8$ représente un alkyle contenant jusqu'à 6 atomes de carbone ;
- $R_9$ représente un alkyle contenant jusqu'à 6 atomes de carbone non substitué ou substitué par un groupe amino libre ou portant un groupe protecteur ;
- ou $R_8$ et $R_9$ ensemble forment avec l'atome de carbone auquel ils sont reliés un cycloalkyle de 3 à 8 atomes de carbone.

**4.** Procédé selon l'une quelconque des revendications 1 à 3, caractérisé en ce que le dernier acide aminé à coupler est choisi de manière que dans le composé de formule I obtenu, $R_1$ représente l'un des groupes suivants :

$(CH_3)_3C-O-\underset{\underset{O}{\|}}{C}-$

$(CH_3)_2CH-CH_2-\underset{\underset{O}{\|}}{C}-$

$C_6H_5-(CH_2)_a-\underset{\underset{O}{\|}}{C}-$, où à = 0, 1 ou 2

$(C_6H_5-CH_2)_2CH-\underset{\underset{O}{\|}}{C}-$

$C_6H_5-CH_2-O-\underset{\underset{O}{\|}}{C}-$

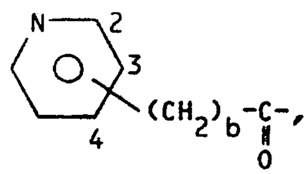
, où b = 0, 1, 2, 3, 4, 5 ou 6

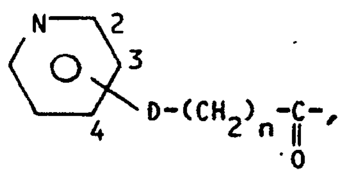
, où D = $-\overset{*}{C}H(OH)-$ ou $-\underset{\underset{O}{\|}}{C}-$

et n = 1, 2, 3, 4 ou 5

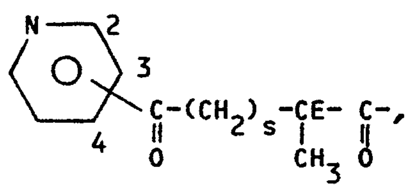
, où E = -H ou $-CH_3$

et s = 1, 2, 3 ou 4

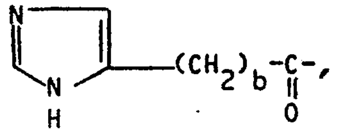 où b = 0, 1, 2, 3, 4, 5 ou 6

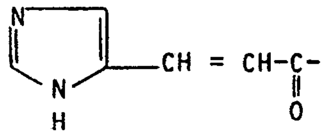

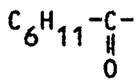

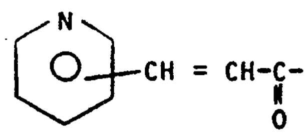

$NH_2CH_2CH_2$-$SO_2$-
Boc$NHCH_2CH_2$-$SO_2$-
Z $NHCH_2CH_2$-$SO_2$-
A - $SO_2$-, où A est un alkyle contenant jusqu'à 6 atomes de carbone, $C_6H_5$ - $(CH_2)_a$ -$SO_2$-, où a = 0, 1 ou 2.

5. Procédé selon l'une quelconque des revendications 1 à 4, caractérisé en ce que le dernier acide aminé à coupler est choisi de manière que, dans le composé de formule I obtenu, $R_1$ représente un groupe choisi parmi les groupes suivants :
   - isovaléryle
   - (pyridyl-2)-4 oxo-4 butyryle
   - (pyridyl-2)-4 hydroxy-4 butyryle
   - (pyridyl-3)-3 propionyle
   - (pyridyl-3)-4 butyryle
   - nicotinoyle
   - benzènesulfonyle.

6. Procédé selon l'une quelconque des revendications 1 à 5, caractérisé en ce que l'on utilise un aminoalcool de formule :

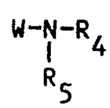

dans laquelle W et $R_4$ sont tels que définis dans la revendication 1 et $R_5$ contient de 1 à 3 groupes hydrxyle.

7. Procédé selon l'une quelconque des revendications 1 à 6, caractérisé en ce que l'un des acides aminés à coupler est choisi de manière que, dans le composé de formule I obtenu, $R_3$ soit tel que le résidu -NH-CH($R_3$)-CO- représente le résidu (tauMe)His dans lequel tauMe désigne l'acide aminé (télé-méthyl) histidine.

8. Procédé selon la revendication 1, caractérisé en ce qu'il consiste à traiter un aminoalcool de formule :

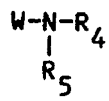

dans laquelle $R_4$ est l'hydrogène, W et $R_5$ sont tels que définis dans la revendication 1 avec le composé de formule H-X'-OH dans laquelle X' est l'un des aminoacides Ile (isoleucine) ou Ala (alanine) et en ce qu'on procède, étape par étape, à l'élongation de la chaîne peptidique par couplage avec des acides aminés ou les fragments suivants, convenablement protégés et choisis de sorte que dans le composé (I) final :

- $R_1$ représente un groupe choisi parmi les groupes suivants : isovaléryle, tert.butyloxycarbonyle, (pyridyl-3)-3 propionyle, (pyridyl-3)-4 butyryle, nicotinoyle ;
- $R_2$ représente un benzyle ;
- $R_3$ représente n-butyle ou un méthyle substitué par un radical hétérocyclique choisi parmi imidazolyl-4, méthyl-1 imidazolyl-4, méthyl-2 imidazolyl-4, pyridyl-3, benzimidazolyl-2 ;
- Q est tel que défini dans la revendication 1.

**9.** Procédé selon la revendication 8, caractérisé en ce que l'on utilise un aminoalcool de formule :

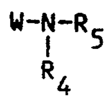

dans laquelle $R_4$ et W sont tels que définis dans la revendication 9 et $R_5$ représente l'un des groupes ci-après :

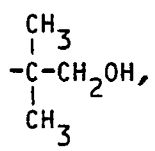 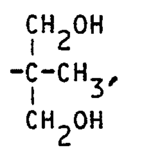 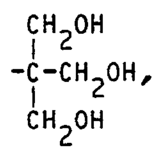 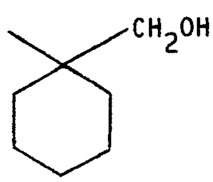

**Claims**

**Claims for the following Contracting States: BE, CH, DE, FR, GB, IT, LI, LU, NL, SE**

**1.** A peptide amino-alcohol derivative of the formula:

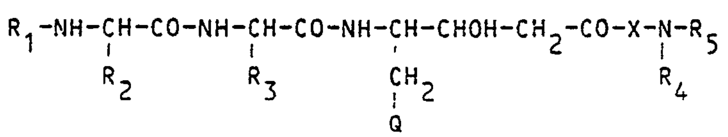 (I)

in which:

- $R_1$ represents an acyl group chosen from the following groups: alkylcarbonyl, alkoxycarbonyl, arylcarbonyl, arylalkylcarbonyl, arylalkoxycarbonyl, heterocyclylcarbonyl, heterocyclylalkylcarbonyl, in which the alkyl group contains 1 to 10 carbon atoms and is optionally substituted by a hydroxyl group, heterocyclylcarbonylalkylcarbonyl, heterocyclylalkenylcarbonyl, cycloalkylcarbonyl, or represents an (alkyl containing up to 6 carbon atoms) sulfonyl group which is unsubstituted or substituted on the alkyl by a free amino group or an amino group carrying a protecting group, or by a phenyl, or represents a phenylsulfonyl group which is unsubstituted or substituted on the phenyl nucleus by an alkyl containing up to 6 carbon atoms;
- $R_2$ represents an alkyl group containing up to 6 carbon atoms which is unsubstituted or substituted by a phenyl, naphthyl, cyclohexyl or pyridyl, or $R_2$ represents a phenyl, naphthyl,

cyclohexyl or pyridyl radical;

- $R_3$ represents hydrogen, an alkenyl containing up to 6 carbon atoms, a phenyl, a naphthyl, a cycloalkyl containing 3 to 6 carbon atoms, a 5-membered or 6-membered monocyclic heterocyclic group which is unsubstituted or substituted with an alkyl containing up to 6 carbon atoms or trifluoromethyl, or alternatively an alkyl containing up to 6 carbon atoms which is unsubstituted or substituted by a free amino group or an amino group carrying a protecting group, by a di(alkyl containing up to 6 carbon atoms)amino group, by a free carboxyl or a carboxyl esterified with an alkyl containing up to 6 carbon atoms or a benzyl, by a free carbamoyl or a carbamoyl substituted with one or two alkyls containing up to 6 carbon atoms or with a phenyl by a hydroxyl, alkoxy group containing up to 6 carbon atoms or benzyloxy group, by a pyridylmethoxy group, by an alkylthio group containing up to 6 carbon atoms, by a (alkyl containing up to 6 carbon atoms) sulfinyl or (alkyl containing up to 6 carbon atoms)sulfonyl group, by a phenyl which is unsubstituted or substituted with a hydroxyl, by a naphthyl, by a cycloalkyl containing from 3 to 6 carbon atoms, by a 5-membered or 6-membered monocyclic heterocyclic group which is unsubstituted or substituted with an alkyl containing up to 6 carbon atoms or a trifluoromethyl, or by a bicyclic heterocyclic group which is unsubstituted or substituted with an alkyl containing up to 6 carbon atoms or a trifluoromethyl;
- $R_4$ represents hydrogen or an alkyl containing up to 6 carbon atoms which is unsubstituted or substituted by an indolyl, pyridyl, imidazolyl or phenyl radical;
- $R_5$ represents an aliphatic or cyclic hydrocarbon group of 3 to 20 carbon atoms which is unsubstituted or substituted by a phenyl, hydroxyphenyl or amino group, the said hydrocarbon group containing at least one hydroxyl group and at least one carbon atom not having a C-H bond;
- Q represents isopropyl, phenyl or cyclohexyl, respectively forming with the radical:

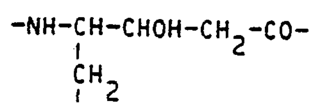

the residue of the amino acid Sta (4-amino-3-hydroxy-6-methylheptanoic acid or statin) ; AHPPA (4-amino-3-hydroxy-5-phenylpentanoic acid) or ACHPA (4-amino-5-cyclohexyl-3-hydroxypentanoic acid);

- X is either a direct bond or the residue of an amino acid such as Ala (alanine), Nva (norvaline), Val (valine), Leu (leucine), Nle (norleucine), Ile (isoleucine), Phg (phenylglycine) Abu ($\alpha$ -aminobutryric acid) or Cpg (cyclopentylglycine);

and also any pharmaceutically acceptable salts of the said peptide amino alcohol with mineral or organic acids or with alkali metals or alkaline earth metals.

**2.** A peptide amino-alcohol derivative as claimed in claim 1, of the formula:

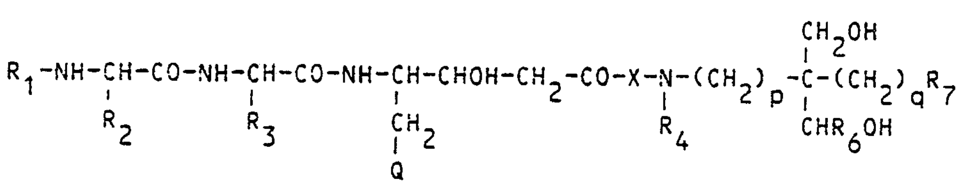

in which $R_1$, $R_2$, $R_3$, Q, X and $R_4$ are as defined in claim 1,

- p represents an integer between 0 and 5;
- q represents an integer between 0 and 5;
- $R_6$ represents hydrogen or an alkyl containing up to 6 carbon atoms; and
- $R_7$ represents hydrogen, a hydroxyl group, an amino group, a carboxyl group, a pyridyl group or a phenyl group which is unsubstituted or substituted by a hydroxyl, with the proviso that if q = 0, $R_7$ is different from hydrogen.

**3.** A peptide amino-alcohol derivative as claimed in claim 1, of the formula:

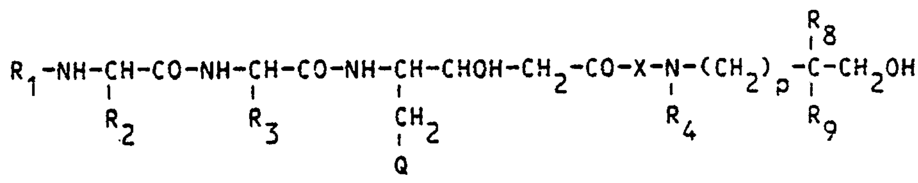

in which $R_1$, $R_2$, $R_3$, Q, X and $R_4$ are as defined in claim 1,

- p represents an integer between 0 and 5;
- $R_8$ represents an alkyl containing up to 6 carbon atoms,
- $R_9$ represents an alkyl containing up to 6 carbon atoms which is unsubstituted or substituted by a free amino group or an amino group carrying a protecting group;
- or $R_8$ and $R_9$ together form a cycloalkyl of 3 to 8 carbon atoms with the carbon atom to which they are joined.

**4.** A peptide amino-alcohol derivative as claimed in anyone of claims 1 to 3, characterized in that $R_1$ represents one of the following groups:

$(CH_3)_3C-O-\underset{O}{\underset{\|}{C}}-$

$(CH_3)_2CH-CH_2-\underset{O}{\underset{\|}{C}}-$

$C_6H_5-(CH_2)_a-\underset{O}{\underset{\|}{C}}-$, in which a = 0, 1 or 2

$(C_6H_5-CH_2)_2CH-\underset{O}{\underset{\|}{C}}-$

$C_6H_5-CH_2-O-\underset{O}{\underset{\|}{C}}-$

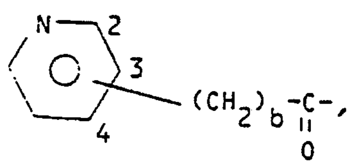, in which b = 0,1,2,3,4, 5 or 6

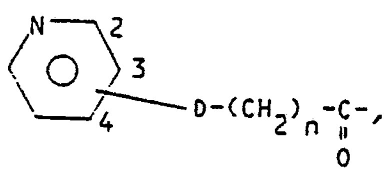, in which D = $-\underset{OH}{\underset{|}{\overset{*}{C}H}}-$ or $-\underset{O}{\underset{\|}{C}}-$

and n = 1, 2, 3, 4 or 5

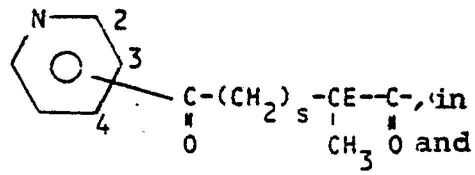 in which E = -H or $-CH_3$ and s = 1, 2, 3 or 4

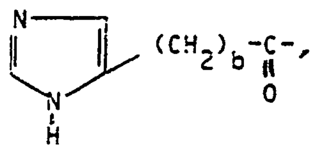, in which b = 0,1,2,3,4,5 or 6

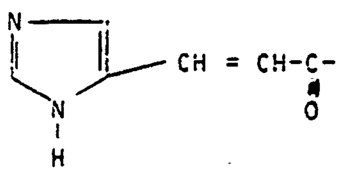

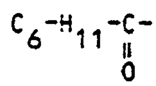

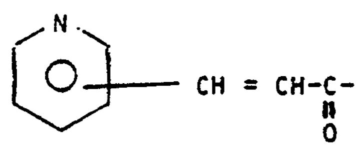

$NH_2CH_2CH_2-SO_2^-$

$BocNHCH_2CH_2-SO_2^-$

$Z-NHCH_2CH_2-SO_2^-$

$A-SO_2-$, in which A is an alkyl containing up to 6 carbon atoms;

$C_6H_5-(CH_2)_a-SO_2$ , in which a = 0, 1 or 2 or one of any salts of the said peptide amino alcohols with pharmaceutically acceptable mineral or organic acids.

**5.** A peptide amino-alcohol derivative as claimed in anyone of claims 1 to 3, characterized in that $R_1$ represents a group chosen from the following groups:

- isovaleryl
- 4-(pyridin-2-yl)-4-oxobutyryl
- (pyridin-2-yl)-4-hydroxybutyryl
- 3-(pyridin-3-yl)propionyl
- 4-(pyridin-3-yl)butyryl
- nicotinoyl

- benzenesulfonyl.

6. A peptide amino-alcohol derivative as claimed in anyone of claims 1 to 5, characterized in that $R_5$ contains from 1 to 3 hydroxyl groups.

7. A peptide amino-alcohol derivative as claimed in anyone of claims 1 to 6, characterized in that $R_3$ is such that the residue -NH-CH($R_3$)-CO- represents the residue (tauMe) His.

8. A peptide amino-alcohol derivative of the formula:

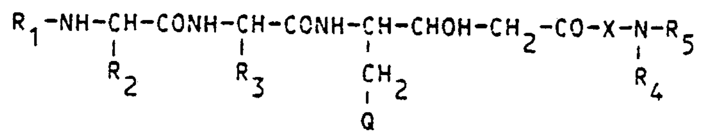

(I)

in which:
- $R_1$ represents a group chosen from the following groups: isovaleryl, tert.-butoxycarbonyl, 3-(pyridin-3-yl)propionyl, 4-(pyridin-3-yl)butyryl, nicotinoyl;
- $R_2$ represents a benzyl;
- $R_3$ represents n-butyl or a methyl substituted by a heterocyclic radical chosen from imidazol-4-yl, 1-methyl-imidazol-4-yl, 2-methylimidazol-4-yl, pyridin-3-yl, benzimidazol-2-yl;
- $R_4$ is hydrogen;
- Q represents isopropyl, phenyl or cyclohexyl, respectively forming with the radical:

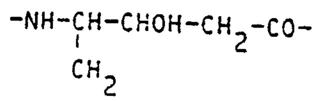

the residue of the amino acid Sta(4-amino-3-hydroxy-6-methylheptanoic acid or statin), AHPPA (4-amino-3-hydroxy-5-phenylheptanoic acid) or ACHPA (4-amino-5-cyclohexyl-3-hydroxypentanoic acid);
- X represents the residue of one of the amino acids Ile (isoleucine) or Ala (alanine);
- $R_5$ represents an aliphatic or cyclic hydrocarbon group of 3 to 20 carbon atoms which is unsubstituted or substituted by a phenyl or hydroxyphenyl or amino group, the said hydrocarbon group simultaneously containing at least one hydroxyl group and at least one carbon atom not having a C-H bond; and also any pharmaceutically acceptable salts of the said peptide amino alcohol with mineral or organic acids or with alkali metals or alkaline earth metals.

9. A peptide amino-alcohol derivative as claimed in claim 8, in which $R_5$ represents a group chosen from:

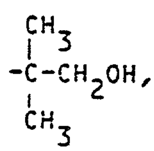, 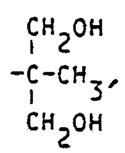, 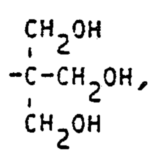, 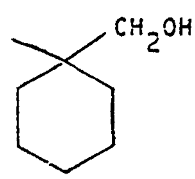

10. A process for the preparation of the peptide amino-alcohol derivatives as claimed in claim 1, characterized in that an amino alcohol of the formula:

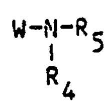

in which W is a protecting group or hydrogen and $R_4$, $R_5$ are as defined in claim 1, and in which the hydroxyl groups are optionally protected, is treated with the alkyl ester (containing up to 6 carbon atoms) of the amino acid of the formula:

protected H-X'-OH or 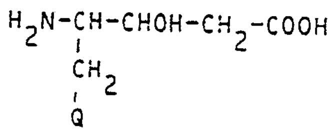

in which X' has the definition given above for X in claim 1, but X' is other than a direct bond, and Q is as defined above, the peptide chain is then lengthened in a stepwise fashion by coupling with the next, appropriately protected amino acids or fragments, the various operations being carried out using either an activated ester of the amino acid or fragment to be coupled or the N-protected amino acid in the presence of DCCI, and the N-protecting groups being cleaved after each coupling, either by hydrogenolysis or by hydrolysis in a strong acid medium, and, if appropriate, the hydroxyl groups of the resulting amino alcohol are deprotected by acid hydrolysis and, if appropriate, the product obtained is converted to one of its pharmaceutically acceptable salts with mineral or organic acids or with alkali metals or alkaline earth metals.

**11.** Drugs, characterized in that they contain, as an active ingredient, a product as claimed in claim 1.

**12.** Drugs as claimed in claim 11, to be specially used for the treatment of hypertension, characterized in that they contain from 1 to 1,000 mg of a product as claimed in claim 1, per dosage unit, mixed with a pharmaceutical excipient.

**Claims for the following Contracting State: AT**

**1.** A process for the preparation of peptide amino-alcohol derivatives of the formula:

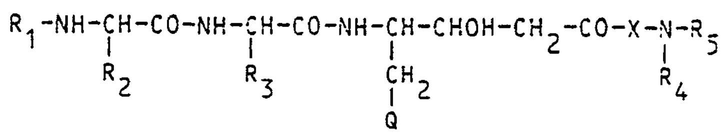

(I)

in which:

- $R_1$ represents an acyl group chosen from the following groups: alkylcarbonyl, alkoxycarbonyl, arylcarbonyl, arylalkylcarbonyl, arylalkoxycarbonyl, heterocyclylcarbonyl, heterocyclylalkylcarbonyl, in which the alkyl group contains 1 to 10 carbon atoms and is optionally substituted by a hydroxyl group, heterocyclylcarbonylalkylcarbonyl, heterocyclylalkenylcarbonyl, cycloalkylcarbonyl, or represents an (alkyl containing up to 6 carbon atoms)sulfonyl group which is unsubstituted or substituted on the alkyl by a free amino group or an amino group carrying a protecting group, or by a phenyl, or represents a phenylsulfonyl group which is unsubstituted or substituted on the phenyl nucleus by an alkyl containing up to 6 carbon atoms;
- $R_2$ represents an alkyl group containing up to 6 carbon atoms which is unsubstituted or substituted by a phenyl, naphthyl, cyclohexyl or pyridyl, or $R_2$ represents a phenyl, naphthyl, cyclohexyl or pyridyl radical;
- $R_3$ represents hydrogen , an alkenyl containing up to 6 carbon atoms, a phenyl, a naphthyl, a cycloalkyl containing 3 to 6 carbon atoms, a 5-membered or 6-membered monocyclic heterocyclic group which is unsubstituted or substituted with an alkyl containing up to 6 carbon atoms or trifluoromethyl, or alternatively an alkyl containing up to 6 carbon atoms which is unsubstituted or substituted by a free amino group or an amino group carrying a protecting group, by a di(alkyl containing up to 6 carbon atoms)amino group, by a free carboxyl or a carboxyl esterified with an alkyl containing up to 6 carbon atoms or a benzyl, by a free carbamoyl or a carbamoyl substituted with one or two alkyls containing up to 6 carbon atoms or with a phenyl, by a hydroxyl, alkoxy group containing up to 6 carbon atoms or benzyloxy group, by a pyridylmethoxy group, by an alkylthio group containing up to 6 carbon atoms, by a (alkyl

containing up to 6 carbon atoms)sulfinyl or (alkyl containing up to 6 carbon atoms)sulfonyl group, by a phenyl which is unsubstituted or substituted with a hydroxyl, by a naphthyl, by a cycloalkyl containing from 3 to 6 carbon atoms, by a 5-membered or 6-membered monocyclic heterocyclic group which is unsubstituted or substituted with an alkyl containing up to 6 carbon atoms or a trifluoromethyl, or by a bicyclic heterocyclic group which is unsubstituted or substituted with an alkyl containing up to 6 carbon atoms or a trifluoromethyl;

- $R_4$ represents hydrogen or an alkyl containing up to 6 carbon atoms which is unsubstituted or substituted by an indolyl, pyridyl, imidazolyl or phenyl radical;
- $R_5$ represents an aliphatic or cyclic hydrocarbon group of 3 to 20 carbon atoms which is unsubstituted or substituted by a phenyl, hydroxyphenyl or amino group, the said hydrocarbon group containing at least one hydroxyl group and at least one carbon atom not having a C-H bond;
- Q represents isopropyl, phenyl or cyclohexyl, respectively forming with the radical:

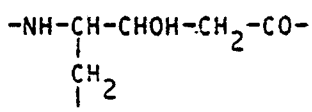

the residue of the amino acid Sta (4-amino-3-hydroxy-6-methylheptanoic acid or statin) ; AHPPA (4-amino-3-hydroxy-5-phenylpentanoic acid) or ACHPA (4-amino-5-cyclohexyl-3-hydroxypentanoic acid);

- X is either a direct bond or the residue of an amino acid such as Ala (alanine), Nva (norvaline), Val (valine), Leu (leucine), Nle (norleucine), Ile (isoleucine), Phg (phenylglycine), Abu ($\alpha$ -aminobutryric acid) or Cpg (cyclopentylglycine).

and also any pharmaceutically acceptable salts of the said peptide amino alcohol with mineral or organic acids or with alkali metals or alkaline earth metals, characterized in that it consists in treating an amino alcohol of the formula:

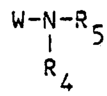

in which W is a protecting group or hydrogen and $R_4$ and $R_5$ are as defined above, and in which the hydroxyl groups are optionally protected, with the alkyl ester (containing up to 6 carbon atoms) of the amino acid of the formula:

protected 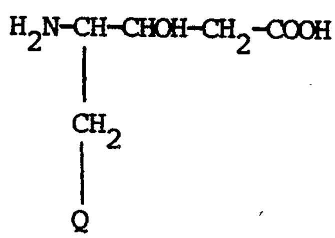 or H-X'-OH

in which X' has the definition given above for X, but X' is other than a direct bond, and Q is as defined above, the peptide chain is then lengthened in a stepwise fashion by coupling with the next, appropriately protected amino acids or fragments, the various operations being carried out using either an activated ester of the amino acid or fragment to be coupled or the N-protected amino acid in the presence of DCCI, and the N-protecting groups being cleaved after each coupling, in deprotecting, if appropriate, the hydroxyl groups of the resulting amino alcohol and in converting, if appropriate, the product obtained to one of its pharmaceutically acceptable salts.

2. A process as claimed in claim 1, characterized in that there is used an amino alcohol of the formula:

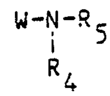

in which $R_4$ and W are as defined in claim 1 and $R_5$ corresponds to the formula:

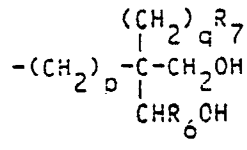

in which:

- p represents an integer between 0 and 5;
- q represents an integer between 0 and 5;
- $R_6$ represents hydrogen or an alkyl containing up to 6 carbon atoms;
- $R_7$ represents hydrogen, a hydroxyl group, an amino group, a carboxyl group, a pyridyl group or a phenyl group which is unsubstituted or substituted by a hydroxyl, with the proviso that if $q = 0$, $R_7$ is different from hydrogen.

**3.** A process as claimed in claim 1, characterized in that there is used an amino alcohol of the formula:

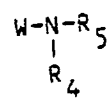

in which $R_4$ and W are as defined in claim 1 and $R_5$ is the group of the formula:

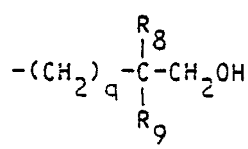

in which

- p represents an integer between 0 and 5;
- $R_8$ represents an alkyl containing up to 6 carbon atoms ;
- $R_9$ represents an alkyl containing up to 6 carbon atoms which is unsubstituted or substituted by a free amino group or an amino group carrying a protecting group;
- or $R_8$ and $R_9$ together form a cycloalkyl of 3 to 8 carbon atoms with the carbon atom to which they are joined.

**4.** A process as claimed in one of claims 1 to 3, characterized in that the last amino acid to be coupled is chosen so that in the resulting compound of the formula I, $R_1$ represents one of the following groups:

$(CH_3)_3C-O-\underset{\underset{O}{\|}}{C}-$

$(CH_3)_2CH-CH_2-\underset{\underset{O}{\|}}{C}-$

$C_6H_5-(CH_2)_a-\underset{\underset{O}{\|}}{C}-$, in which a = 0, 1 or 2

$(C_6H_5-CH_2)_2CH-\underset{\underset{O}{\|}}{C}-$

$C_6H_5-CH_2-O-\underset{\underset{O}{\|}}{C}-$

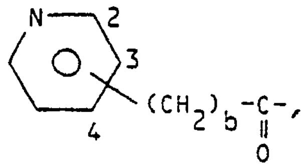 in which b = 0, 1, 2, 3, 4,5 or 6

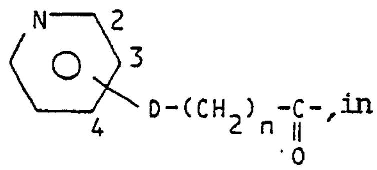 in which D = 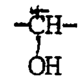 or 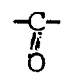

and n = 1, 2, 3, 4 ou 5

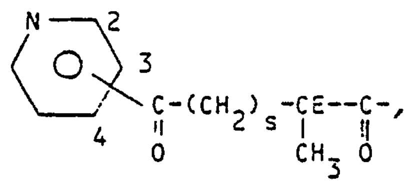 in which E = -H or $-CH_3$ and s = 1, 2, 3 or 4

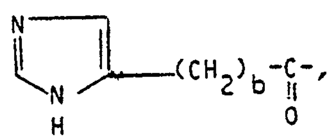 in which b = 0,1,2,3,4,5 or 6

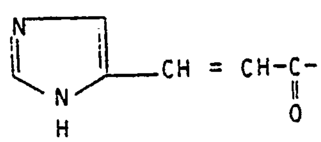

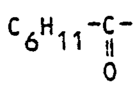

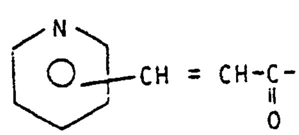

$NH_2CH_2CH_2-SO_2-$
$BooNHCH_2CH_2-SO_2-$
$Z\ NHCH_2CH_2-SO_2-$
$A-SO_2-$, in which A is an alkyl containing up to 6 carbon atoms $C_6H_5-(CH_2)_a-SO_2-$, in which a = 0, 1 or 2.

5. A process as claimed in one of claims 1 to 4, characterized in that the last amino acid to be coupled is chosen so that in the resulting compound of the formula I, $R_1$ represents a group chosen from the following groups:
   - isovaleryl
   - 4-(pyridin-2-yl)-4-oxobutyryl
   - 4-(pyridin-2-yl)-4-hydroxybutyryl
   - 3-(pyridin-3-yl)propionyl

- 4-(pyridin-3-yl)butyryl
- nicotinoyl
- benzenesulfonyl.

**6.** A process as claimed in one of claims 1 to 5, characterized in that there is used an amino alcohol of the formula:

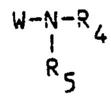

in which W and $R_4$ are as defined in claim 1 and $R_5$ contains from 1 to 3 hydroxyl groups.

**7.** A process as claimed in one of claims 1 to 6, characterized in that one of the amino acids to be coupled is chosen so that, in the resulting compound of the formula I, $R_3$ is such that the residue NH-CH($R_3$)-CO- represents the residue (tauMe)His in which tauMe means the amino acid (tele-methyl)-histidine.

**8.** A process as claimed in claim 1, characterized in that it consists in treating an amino alcohol of the formula:

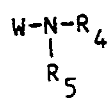

in which $R_4$ is hydrogen, W and $R_5$ are as defined in claim 1 with the compound of the formula H-X'-OH in which X' is one of the amino acid Ile (isoleucine) or Ala (alanine) and in that the peptide chain is lengthened in a stepwise fashion by coupling with the next, appropriately protected amino acids or fragments, chosen so that in the final compound (I):

- $R_1$ represents a group chosen from the following groups: isovaleryl, tert.-butoxycarbonyl, 3-(pyridin-3-yl)propionyl, 4-(pyridin-3-yl)butyryl, nicotinoyl;
- $R_2$ represents a benzyl;
- $R_3$ represents n-butyl or a methyl substituted by a heterocyclic radical chosen from imidazol-4-yl, 1-methyl-imidazol-4-yl, 2-methylimidazol-4-yl, pyridin-3-yl and benzimidazol-2-yl;
- Q is as defined in claim 1.

**9.** A process as claimed in claim. 8, characterized in that there is used an amino alcohol of the formula:

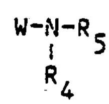

in which $R_4$ and W are as defined in claim 9 and $R_5$ represents a group chosen from:

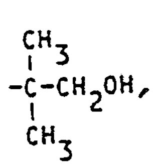
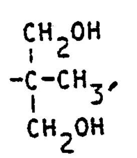
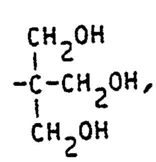
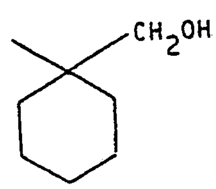

**Patentansprüche**

**Patentansprüche für folgende Vertragsstaaten: BE, CH, DE, FR, GB, IT, LI, LU, NL, SE**

1. Peptidaminoalkoholderivat der Formel

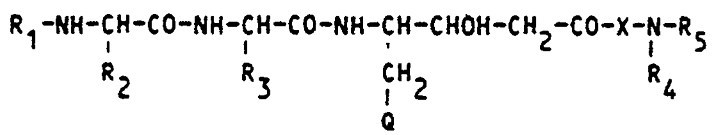 (I)

worin:

- $R_1$ eine Acylgruppe, ausgewählt aus den Gruppen Alkylcarbonyl, Alcoxycarbonyl, Arylcarbonyl, Arylalkylcarbonyl, Arylalcoxycarbonyl, Heterocyclylcarbonyl, Heterocylcylalkylcarbonyl, in der die Alkylgruppe 1 bis 10 Kohlenstoffatome enthält und gegebenenfalls substituiert ist durch eine Hydroxygruppe, Heterocyclylcarbonylalkylcarbonyl, Heterocyclylalkenylcarbonyl, Cycloalkylcarbonyl, oder eine nicht substituierte oder am Alkyl durch eine freie oder eine Schutzgruppe tragende Aminogruppe oder durch ein Phenyl substituierte (Alkyl mit bis zu 6 Kohlenstoffatomen)-sulfonylgruppe; oder eine nicht substituierte oder am Phenylkern durch ein Alkyl mit bis zu 6 Kohlenstoffatomen substituierte Phenylsulfonylgruppe darstellt;
- $R_2$ eine nicht substituierte oder durch ein Phenyl, Naphthyl, Cyclohexyl oder Pyridyl substituierte Alkylgruppe mit bis zu 6 Kohlenstoffatomen darstellt, oder $R_2$ für einen Phenyl-, Naphthyl-, Cyclohexyl- oder Pyridylrest steht;
- $R_3$ Wasserstoff, ein Alkenyl mit bis zu 6 Kohlenstoffatomen, Phenyl, Naphthyl, Cycloalkyl mit 3 bis 6 Kohlenstoffatomen, eine nicht substituierte oder durch ein Alkyl mit bis zu 6 Kohlenstoffatomen oder Trifluormethyl substituierte monocyclische heterocyclische Gruppe mit 5 oder 6 Ketten darstellt; oder aber ein Alkyl mit bis zu 6 Kohlenstoffatomen, nicht substituiert oder substituiert durch eine freie oder eine Schutzgruppe tragende Aminogruppe, durch eine Di(Alkyl mit bis zu 6 Kohlenstoffatomen)-aminogruppe, durch ein freies oder mit einem Alkyl mit bis zu 6 Kohlenstoffatomen oder Benzyl verestertes Carboxyl, durch ein freies oder mit einem oder zwei Alkylen mit bis zu 6 Kohlenstoffatomen oder mit Phenyl substituiertes Carbamoyl, durch eine Hydroxygruppe, Alkoxy mit bis zu 6 Kohlenstoffatomen oder Benzyloxy, durch eine Gruppe Pyridylmethyloxy, durch eine Gruppe Alkylthio mit bis zu 6 Kohlenstoffatomen, (Alkyl mit bis zu 6 Kohlenstoffatomen)-sulfinyl oder (Alkyl mit bis zu 6 Kohlenstoffatomen)-sulfonyl, durch ein nicht substituiertes oder mit einem Hydroxyl substituiertes Phenyl, durch ein Naphthyl, durch ein Cycloalkyl mit 3 bis 6 Kohlenstoffatomen, durch eine nicht substituierte oder mit einem Alkyl mit bis zu 6 Kohlenstoffatomen oder einem Trifluormethyl substituierte monocyclische heterocyclische Gruppe mit 5 oder 6 Ketten oder durch eine nicht substituierte oder mit einem Alkyl mit bis zu 6 Kohlenstoffatomen oder Trifluormethyl substituierte bicyclische heterocyclische Gruppe;
- $R_4$ Wasserstoff oder ein nicht substituiertes oder mit einem Indolyl-, Pyridyl-, Imidazolyl- oder Phenylrest substituiertes Alkyl mit bis zu 6 Kohlenstoffatomen darstellt;
- $R_5$ eine nicht substituierte oder durch eine Phenylgruppe, Hydroxyphenyl oder Amino substituierte aliphatische oder cyclische Kohlenwasserstoffgruppe mit 3 bis 20 Kohlenstoffatomen darstellt, welche Kohlenwasserstoffgruppe mindestens eine Hydroxylgruppe und mindestens ein Kohlenstoffatom ohne C-H-Bindung enthält;
- Q für Isopropyl, Phenyl oder Cyclohexyl steht, das mit der Gruppe

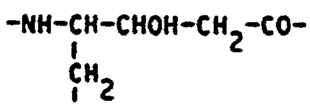

den Aminosäurerest Sta (4-Amino-3-hydroxy-6-methylheptansäure oder Statin); AHPPA (4-Amino-3-hydroxy-5-phenyl-pentansäure) bzw. ACHPA (4-Amino-5-cyclohexyl-3-hydroxy-pentansäure) bildet;
- X entweder eine direkte Bindung oder der Rest einer Aminosäure, wie Ala (Alanin), Nva (Norvalin), Val (Valin), Leu (Leucin), Nle (Norleucin), Ile (Isoleucin), Phg (Phenylglycin), Abu ($\alpha$-Aminobuttersäure), Cpg (Cyclopentylglycin); ist;

sowie die möglichen pharmazeutisch akzeptablen Salze des Peptidaminoalkohols mit Mineral- oder

organischen Säuren oder mit Alkali- oder Erdalkalimetallen.

2. Peptpidaminoalkoholderivat nach Anspruch 1 der Formel

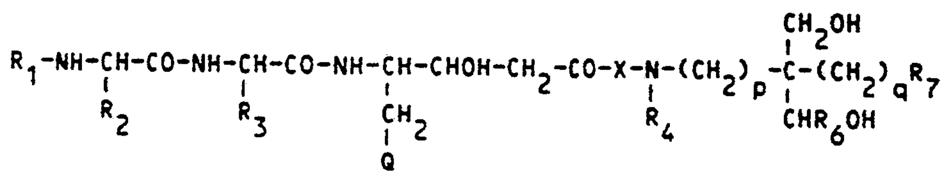

worin $R_1$, $R_2$, $R_3$, Q, x und $R_4$ wie in Anspruch 1 definiert sind,

- p für eine ganze Zahl zwischen 0 und 5 steht;
- q für eine ganze Zahl zwischen 0 und 5 steht;
- $R_6$ für Wasserstoff oder ein Alkyl mit bis zu 6 Kohlenstoffatomen steht und
- $R_7$ Wasserstoff, eine Hydroxylgruppe, eine Aminogruppe, eine Carboxygruppe, eine Pyridylgruppe oder eine Phenylgruppe, nicht substituiert oder durch ein Hydroxyl substituiert, darstellt, mit der Maßgabe, daß $R_7$ nicht Wasserstoff ist, wenn q = 0.

3. Peptidaminoalkohohlderivat nach Anspruch 1 der Formel

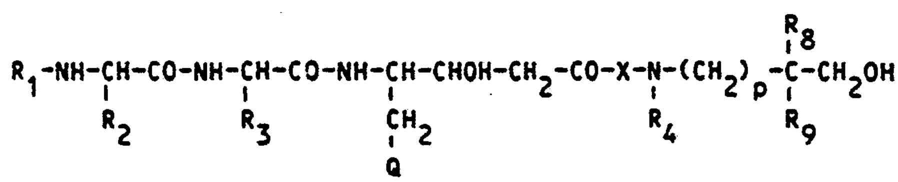

worin $R_1$, $R_2$, $R_3$, Q, X und $R_4$ wie in Anspruch 1 definiert sind,

- p für eine ganze Zahl zwischen 0 und 5 steht;
- $R_8$ ein Alkyl mit bis zu 6 Kohlenstoffatomen darstellt;
- $R_9$ ein nicht substituiertes oder durch eine freie oder eine Schutzgruppe tragende Aminogruppe substituiertes Alkyl mit bis zu 6 Kohlenstoffatomen darstellt;
- oder $R_8$ und $R_9$ zusammen mit dem Kohlenstoffatom, an das sie gebunden sind, ein Cycloalkyl mit 3 bis 8 Kohlenstoffatomen bilden.

4. Peptidaminoalkoholderivat nach einem der Ansprüche 1 bis 3, dadurch gekennzeichnet, daß $R_1$ eine der folgenden Gruppen repräsentiert:

$$(CH_3)_3C-O-\underset{\substack{\| \\ O}}{C}-$$

$$(CH_3)_2CH-CH_2-\underset{\substack{\| \\ O}}{C}-$$

$$C_6H_5-(CH_2)_a-\underset{\substack{\| \\ O}}{C}-,$$ worin a = 0, 1 oder 2

$$(C_6H_5-CH_2)_2CH-\underset{\substack{\| \\ O}}{C}-$$

$$C_6H_5-CH_2-O-\underset{\substack{\| \\ O}}{C}-$$

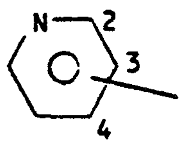

$$(CH_2)_b-\underset{\substack{\| \\ O}}{C}-,$$ worin b = 0, 1, 2, 3, 4, 5 oder 6

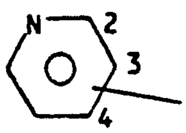

$$D-(CH_2)_n-\underset{\substack{\| \\ O}}{C}-,$$ worin $D = -\underset{\substack{| \\ OH}}{\overset{*}{C}H}-$ oder $-\underset{\substack{\| \\ O}}{C}-$

und n = 1, 2, 3, 4 oder 5

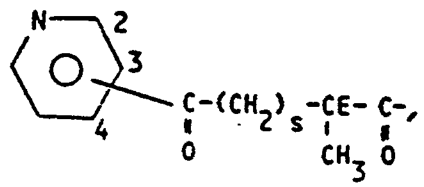 worin E = -H oder $-CH_3$ und s = 1, 2, 3 oder 4

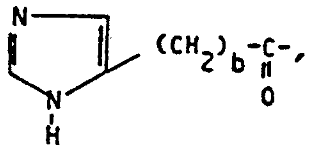 worin b = 0, 1, 2, 3, 4, 5 oder 6

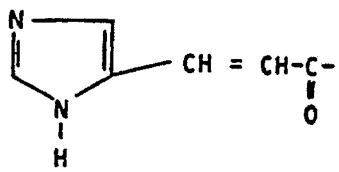

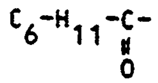

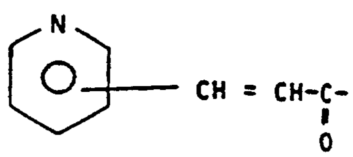

$NH_2CH_2CH_2-SO_2-$

$BocNHCH_2CH_2-SO_2-$

$Z-NHCH_2CH_2-SO_2-$

$A-SO_2-$, worin A ein Alkyl mit bis zu 6 Kohlenstoffatomen ist;
$C_6H_5-(CH_2)_a-SO_2$, worin a = 0, 1 oder 2,
oder eines der möglichen pharmazeutisch akzeptablen Salze der Peptidaminoalkohole von Mineral- oder organischen Säuren.

5. Peptidominoalkoholderivat nach einem der Ansprüche 1 bis 3, dadurch gekennzeichnet, daß $R_1$ eine Gruppe darstellt, ausgewählt aus den folgenden Gruppen:
   - Isovaleryl
   - 4-(2-Pyridyl)-4-oxo-butyryl
   - 4-(2-Pyridyl)-4-hydroxy-butyryl
   - 3-(3-Pyridyl)-propionyl
   - 4-(3-Pyridyl)-butyryl
   - Nicotinoyl

- Benzolsulfonyl.

6. Peptidaminoalkoholderivat nach einem der Ansprüche 1 bis 5, dadurch gekennzeichnet, daß $R_5$ 1 bis 3 Hydroxylgruppen enthält.

7. Peptidaminoalkoholderivat nach einem der Ansprüche 1 bis 6, dadurch gekennzeichnet, daß $R_3$ derart ist, daß der Rest -NH-CH($R_3$)CO- den Rest (tauMe)His darstellt.

8. Peptidaminoalkoholderivat der Formel

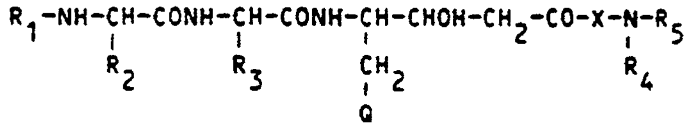

(I)

in welcher:

- $R_1$ eine Gruppe darstellt, ausgewählt aus den folgenden Gruppen: Isovaleryl, tert.Butyloxycarbonyl, 3-(3-Pyridyl)-propionyl, 4-(3-Pyridyl)-butyryl, Nicotinoyl;
- $R_2$ Benzyl darstellt;
- $R_3$ n-Butyl oder ein durch einen heterocyclischen Rest, ausgewählt aus 4-Imidazolyl, 1-Methyl-4-imidazolyl, 2-Methyl-4-imidazolyl, 3-Pyridyl, Benzimidazol-2-yl, substituiertes Methyl darstellt;
- $R_4$ für Wasserstoff steht;
- Q Isopropyl, Phenyl oder Cyclohexyl darstellt, das mit der Gruppe

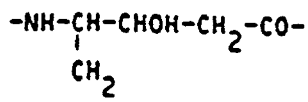

den Aminosäurerest Sta (4-Amino-3-hydroxy-6-methylheptansäure oder Statin); AHPPA (4-Amino-3-hydroxy--5-phenyl-pentansäure) bzw. ACHPA (4-Amino-5--cyclohexyl-3-hydroxy-pentansäure) bildet;
- X den Rest einer der Aminosäuren Ile (Isoleucin) oder Ala (Alanin) darstellt;
- $R_5$ eine aliphatische oder cyclische Kohlenwasserstoffgruppe mit 3 bis 20 Kohlenstoffatomen, nicht substituiert oder durch eine Phenylgruppe, Hydroxyphenyl, Amino substituiert, darstellt, welche Kohlenwasserstoffgruppe gleichzeitig mindestens eine Hydroxylgruppe und mindestens ein Kohlenstoffatom ohne C-H-Bindung enthält

sowie die möglichen pharmazeutisch akzeptablen Salze des Peptidaminoalkohols mit Mineral- oder organischen Säuren oder mit Alkali- oder Erdalkalimetallen.

9. Peptidaminoalkoholderivat nach Anspruch 8, worin $R_5$ eine Gruppe ausgewählt aus

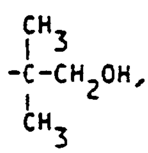
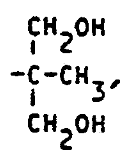
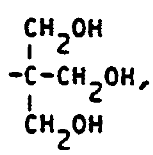
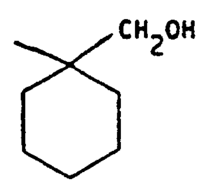

darstellt.

10. Verfahren zur Herstellung der Peptidaminoalkoholderivate nach Anspruch 1, dadurch gekennzeichnet, daß man einen Aminoalkohol der Formel

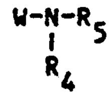

worin W eine Schutzgruppe oder Wasserstoff bedeutet und $R_4$, $R_5$ wie in Anspruch 1 definiert sind und wobei die Hydroxylgruppen gegebenenfalls geschützt sind, mit dem Alkylester (mit bis zu 6 Kohlenstoffatomen) der Aminosäure der Formel

$H-X'-OH$ oder 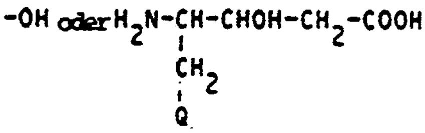 geschützt,

worin X' die in Anspruch 1 für X angegebene Bedeutung hat, X' aber keine direkte Bindung ist, und Q wie oben definiert ist, behandelt, man danach schrittweise die Verlängerung der Peptidkette durch Kopplung mit den folgenden Aminosäuren oder deren Fragmenten, zweckmäßig geschützt, vornimmt, wobei die verschiedenen Verfahrensschritte unter Verwendung entweder eines aktivierten Esters der zu koppelnden Aminosäure oder der N-geschützten Aminosäure in Gegenwart von DCCI ausgeführt werden, die N-Schutzgruppen nach jeder Kopplung entweder durch Hydrogenolyse oder durch Hydrolyse in stark saurem Milieu abgespalten werden, und man gegebenenfalls die Hydroxylgruppen des so erhaltenen Aminoalkohols durch saure Hydrolyse entschützt; und man notwendigenfalls das so erhaltene Produkt in eines seiner Salze von Mineral- oder organischen Säuren oder Alkali- oder Erdalkalimetallen überführt.

11. Medikamente, dadurch gekennzeichnet, daß sie als aktives Ingrediens ein Produkt nach Anspruch 1 enthalten.

12. Medikamente nach Anspruch 11 zur Verwendung insbesondere bei der Behandlung des arteriellen Drucks, dadurch gekennzeichnet, daß sie 1 bis 1000 mg Produkt nach Anspruch 1 pro Dosiseinheit in Mischung mit einem pharmazeutischen Exzipienten enthalten.

**Patentansprüche für folgenden Vertragsstaat: AT**

1. Verfahren zur Herstellung von Peptidaminoalkoholderivaten der Formel

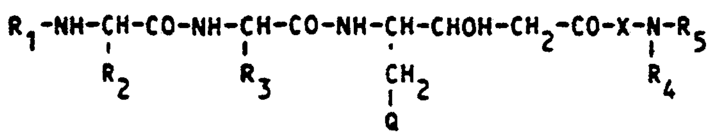 (I)

worin:

- $R_1$ eine Acylgruppe, ausgewählt aus den Gruppen Alkylcarbonyl, Alcoxycarbonyl, Arylcarbonyl, Arylalkylcarbonyl, Arylalcoxycarbonyl, Heterocyclylcarbonyl, Heterocylcylalkylcarbonyl, in der die Alkylgruppe 1 bis 10 Kohlenstoffatome enthält und gegebenenfalls substituiert ist durch eine Hydroxygruppe, Heterocyclylcarbonylalkylcarbonyl, Heterocyclylalkenylcarbonyl, Cycloalkylcarbonyl, oder eine nicht substituierte oder am Alkyl durch eine freie oder eine Schutzgruppe tragende Aminogruppe oder durch ein Phenyl substituierte (Alkyl mit bis zu 6 Kohlenstoffatomen)-sulfonylgruppe; oder eine nicht substituierte oder am Phenylkern durch ein Alkyl mit bis zu 6 Kohlenstoffatomen substituierte Phenylsulfonylgruppe darstellt;
- $R_2$ eine nicht substituierte oder durch ein Phenyl, Naphthyl, Cyclohexyl oder Pyridyl substituierte Alkylgruppe mit bis zu 6 Kohlenstoffatomen darstellt, oder $R_2$ für einen Phenyl-, Naphthyl-, Cyclohexyl- oder Pyridylrest steht;
- $R_3$ Wasserstoff, ein Alkenyl mit bis zu 6 Kohlenstoffatomen, Phenyl, Naphthyl, Cycloalkyl mit 3 bis 6 Kohlenstoffatomen, eine nicht substituierte oder durch ein Alkyl mit bis zu 6 Kohlenstoffatomen oder Trifluormethyl substituierte monocyclische heterocyclische Gruppe mit 5 oder 6 Ketten

darstellt; oder aber ein Alkyl mit bis zu 6 Kohlenstoffatomen, nicht substituiert oder substituiert durch eine freie oder eine Schutzgruppe tragende Aminogruppe, durch eine Di(Alkyl mit bis zu 6 Kohlenstoffatomen)-aminogruppe, durch ein freies oder mit einem Alkyl mit bis zu 6 Kohlenstoffatomen oder Benzyl verestertes Carboxyl, durch ein freies oder mit einem oder zwei Alkylen mit bis zu 6 Kohlenstoffatomen oder mit Phenyl substituiertes Carbamoyl, durch eine Hydroxygruppe, Alkoxy mit bis zu 6 Kohlenstoffatomen oder Benzyloxy, durch eine Gruppe Pyridylmethyloxy, durch eine Gruppe Alkylthio mit bis zu 6 Kohlenstoffatomen, (Alkyl mit bis zu 6 Kohlenstoffatomen)-sulfinyl oder (Alkyl mit bis zu 6 Kohlenstoffatomen)-sulfonyl, durch ein nicht substituiertes oder mit einem Hydroxyl substituiertes Phenyl, durch ein Naphthyl, durch ein Cycloalkyl mit 3 bis 6 Kohlenstoffatomen, durch eine nicht substituierte oder mit einem Alkyl mit bis zu 6 Kohlenstoffatomen oder einem Trifluormethyl substituierte monocyclische heterocyclische Gruppe mit 5 oder 6 Ketten oder durch eine nicht substituierte oder mit einem Alkyl mit bis zu 6 Kohlenstoffatomen oder Trifluormethyl substituierte bicyclische heterocyclische Gruppe;

- $R_4$ Wasserstoff oder ein nicht substituiertes oder mit einem Indolyl-, Pyridyl-, Imidazolyl- oder Phenylrest substituiertes Alkyl mit bis zu 6 Kohlenstoffatomen darstellt;
- $R_5$ eine nicht substituierte oder durch eine Phenylgruppe, Hydroxyphenyl oder Amino substituierte aliphatische oder cyclische Kohlenwasserstoffgruppe mit 3 bis 20 Kohlenstoffatomen darstellt, welche Kohlenwasserstoffgruppe mindestens eine Hydroxylgruppe und mindestens ein Kohlenstoffatom ohne C-H-Bindung enthält;
- Q für Isopropyl, Phenyl oder Cyclohexyl steht, das mit der Gruppe

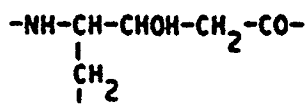

den Aminosäurerest Sta (4-Amino-3-hydroxy-6-methylheptansäure oder Statin); AHPPA (4-Amino-3-hydroxy-5-phenyl-pentansäure) bzw. ACHPA (4-Amino-5-cyclohexyl-3-hydroxy-pentansäure) bildet;
- X entweder eine direkte Bindung oder der Rest einer Aminosäure, wie Ala (Alanin), Nva (Norvalin), Val (Valin), Leu (Leucin), Nle (Norleucin), Ile (Isoleucin), Phg (Phenylglycin), Abu (α-Aminobuttersäure), Cpg (Cyclopentylglycin); ist;

sowie der möglichen pharmazeutisch akzeptablen Salze des Peptidaminoalkohols mit Mineral- oder organischen Säuren oder mit Alkali- oder Erdalkalimetallen, dadurch gekennzeichnet, daß es darin besteht, daß man einen Aminoalkohol der Formel

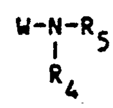

worin W eine Schutzgruppe oder Wasserstoff bedeutet und $R_4$, $R_5$ wie oben definiert sind und wobei die Hydroxylgruppen gegebenenfalls geschützt sind, mit dem Alkylester (mit bis zu 6 Kohlenstoffatomen) der Aminosäure der Formel

$H-X'-OH$ oder 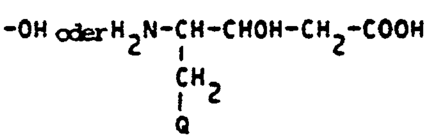 geschützt,

worin X' die oben für X angegebene Bedeutung hat, X' aber keine direkte Bindung ist, und Q wie oben definiert ist, behandelt, man danach schrittweise die Verlängerung der Peptidkette durch Kopplung mit den folgenden Aminosäuren oder deren Fragmenten, zweckmäßig geschützt, vornimmt, wobei die verschiedenen Verfahrensschritte unter Verwendung entweder eines aktivierten Esters der zu koppelnden Aminosäure bzw. des zu koppelnden Fragments oder der N-geschützten Aminosäure in Gegenwart von DCCI ausgeführt werden, man die N-Schutzgruppen nach jeder Kopplung abspaltet, man erforderlichenfalls die Hydroxylgruppen des so erhaltenen Aminoalkohols entschützt, und man gegebenenfalls

das so erhaltene Produkt in eines seiner pharmazeutisch akzeptablen Salze überführt.

2. Verfahren nach Anspruch 1, dadurch gekennzeichnet, daß man einen Aminoalkohol der Formel

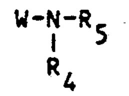

verwendet, worin $R_4$ und W wie in Anspruch 1 definiert sind und $R_5$ der Formel

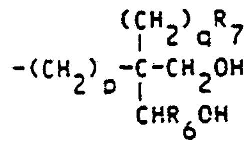

entspricht, worin
- p für eine ganze Zahl zwischen 0 und 5 steht;
- q für eine ganze Zahl zwischen 0 und 5 steht;
- $R_6$ für Wasserstoff oder ein Alkyl mit bis zu 6 Kohlenstoffatomen steht,
- $R_7$ Wasserstoff, eine Hydroxylgruppe, eine Aminogruppe, eine Carboxygruppe, eine Pyridylgruppe oder eine Phenylgruppe, nicht substituiert oder durch ein Hydroxyl substituiert, darstellt, mit der Maßgabe, daß $R_7$ nicht Wasserstoff ist, wenn q = 0.

3. Verfahren nach Anspruch 1, dadurch gekennzeichnet, daß man einen Aminoalkohol der Formel

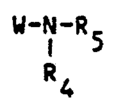

verwendet, worin $R_4$ und W wie in Anspruch 1 definiert sind und $R_5$ der Formel

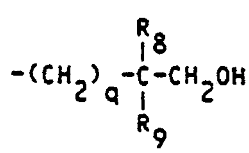

entspricht, worin
- p für eine ganze Zahl zwischen 0 und 5 steht;
- $R_8$ ein Alkyl mit bis zu 6 Kohlenstoffatomen darstellt;
- $R_9$ ein nicht substituiertes oder durch eine freie oder eine Schutzgruppe tragende Aminogruppe substituiertes Alkyl mit bis zu 6 Kohlenstoffatomen darstellt;
- oder $R_8$ und $R_9$ zusammen mit dem Kohlenstoffatom, an das sie gebunden sind, ein Cycloalkyl mit 3 bis 8 Kohlenstoffatomen bilden.

4. Verfahren nach einem der Ansprüche 1 bis 3, dadurch gekennzeichnet, daß die letzte zu koppelnde Aminosäure derart gewählt wird, daß in der erhaltenen Verbindung der Formel I $R_1$ eine der folgenden Gruppen repräsentiert:

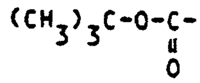

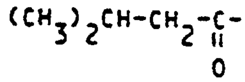

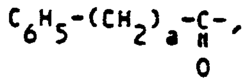 worin a = 0, 1 oder 2

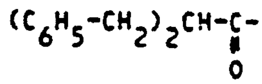

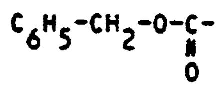

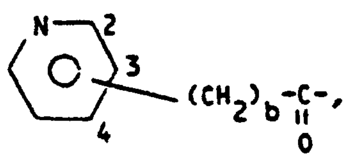 worin b = 0, 1, 2, 3, 4, 5 oder 6

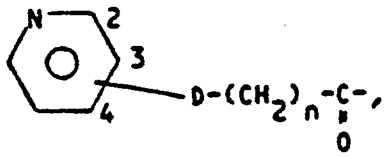

worin D = 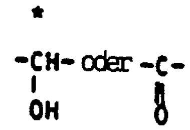

und $n$ = 1, 2, 3, 4 oder 5

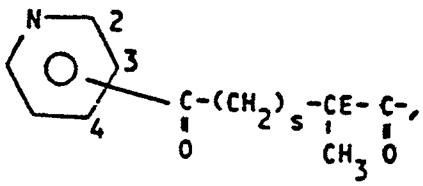 worin E = -H oder $-CH_3$ und s = 1, 2, 3 oder 4

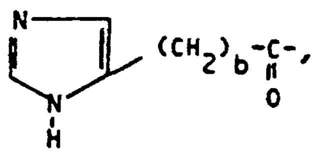 worin b = 0, 1, 2, 3, 4, 5 oder 6

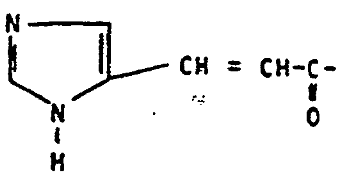

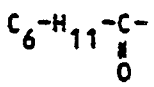

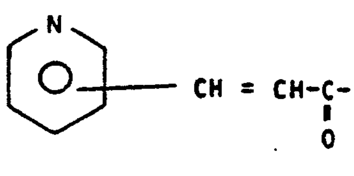

$NH_2CH_2CH_2-SO_2^-$

$BocNHCH_2CH_2-SO_2^-$

$Z-NHCH_2CH_2-SO_2^-$

$A-SO_2-$, worin A ein Alkyl mit bis zu 6 Kohlenstoffatomen ist;
$C_6H_5-(CH_2)_a-SO_2$, worin a = 0, 1 oder 2.

5. Verfahren nach einem der Ansprüche 1 bis 4, dadurch gekennzeichnet, daß die letzte zu koppelnde Aminosäure derart gewählt wird, daß in der erhaltenen Verbindung der Formel I $R_1$ eine Gruppe, ausgewählt aus den folgenden Gruppen, repräsentiert:

- Isovaleryl

- 4-(2-Pyridyl)-4-oxo-butyryl
- 4-(2-Pyridyl)-4-hydroxy-butyryl
- 3-(3-Pyridyl)-propionyl
- 4-(3-Pyridyl)-butyryl
- Nicotinoyl
- Benzolsulfonyl.

6. Verfahren nach einem der Ansprüche 1 bis 5, dadurch gekennzeichnet, daß man einen Aminoalkohol der Formel

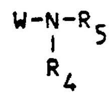

verwendet, worin W und $R_4$ wie in Anspruch 1 definiert sind und $R_5$ 1 bis 3 Hydroxylgruppen enthält.

7. Verfahren nach einem der Ansprüche 1 bis 6, dadurch gekennzeichnet, daß eine der zu koppelnden Aminosäure derart gewählt wird, daß in der erhaltenen Verbindung der Formel I $R_3$ derart ist, daß der Rest -NH-CH($R_3$)-CO- den Rest (tauMe)His darstellt, worin tauMe die Aminosäure (Telemethyl)-histidin bezeichnet.

8. Verfahren nach Anspruch 1, dadurch gekennzeichnet, daß es darin besteht, daß man einen Aminoalkohol der Formel

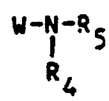

worin $R_4$ Wasserstoff ist, W und $R_5$ wie in Anspruch 1 definiert sind, mit der Verbindung der Formel H-X'-OH, in der X' eine der Aminosäuren Ile (Isoleucin) oder Ala (Alanin) ist, behandelt und man danach schrittweise die Verlängerung der Peptidkette vornimmt, u.zw. durch Kopplung mit folgenden Aminosäuren oder Fragmenten, zweckmäßig geschützt und derart gewählt, daß in der Endverbindung (I):
- $R_1$ eine Gruppe darstellt, ausgewählt aus den folgenden Gruppen: Isovaleryl, tert.Butyloxycarbonyl, 3-(3-Pyridyl)-propionyl, 4-(3-Pyridyl)-butyryl, Nicotinoyl;
- $R_2$ Benzyl darstellt;
- $R_3$ n-Butyl oder ein durch einen heterocyclischen Rest, ausgewählt aus 4-Imidazolyl, 1-Methyl-4-imidazolyl, 2-Methyl-4-imidazolyl, 3-Pyridyl, Benzimidazol-2-yl, substituiertes Methyl darstellt;
- Q wie in Anspruch 1 definiert ist.

9. Verfahren nach Anspruch 8, dadurch gekennzeichnet, daß man einen Aminoalkohol der Formel

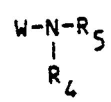

verwendet, worin $R_4$ und W wie in Anspruch 9 definiert sind und $R_5$ eine der nachstehenden Gruppen repräsentiert: